(19) Europäisches Patentamt
European Patent Office
Office européen des brevets

(11) EP 2 804 119 A2

(12) # EUROPEAN PATENT APPLICATION

(43) Date of publication:
**19.11.2014 Bulletin 2014/47**

(51) Int Cl.:
***G06F 19/00*** *(2011.01)*

(21) Application number: **14168538.8**

(22) Date of filing: **15.05.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.05.2013 JP 2013104664**

(71) Applicant: **Hitachi Ltd.**
**Tokyo (JP)**

(72) Inventors:
- **Miyoshi, Toshinori**
**Chiyoda-ku, Tokyo 100-8280 (JP)**
- **Hasegawa, Yasutaka**
**Chiyoda-ku, Tokyo 100-8280 (JP)**
- **Ban, Hideyuki**
**Chiyoda-ku, Tokyo 100-8280 (JP)**
- **Nagasaki, Takeshi**
**Chiyoda-ku, Tokyo 100-8280 (JP)**
- **Shinjo, Hiroshi**
**Chiyoda-ku, Tokyo 100-8280 (JP)**

(74) Representative: **Moore, Graeme Patrick et al**
**Mewburn Ellis LLP**
**33 Gutter Lane**
**London**
**EC2V 8AS (GB)**

(54) **Analysis System and Health Business Support Method**

(57) It is provided an analysis system comprising: a causation/transition structure calculating unit generating a graph structure including a node and a probability variable relating to item, and a probabilistic dependency defined by one of a directed link or an undirected link between the nodes; a node generating unit generating an event space of the nodes; a probability calculating unit calculating a conditional probability of the graph structure; a state transition model reconstructing unit reconstructing a state transition model with a graph structure, an event space and a conditional probability including specified probability variables based on a state transition model; a disease state transition estimating unit estimating a disease state transition probability based on the reconstructed state transition model; and a health guidance supporting unit selecting a subject for health guidance and a content of health guidance based on the estimated disease state transition probability.

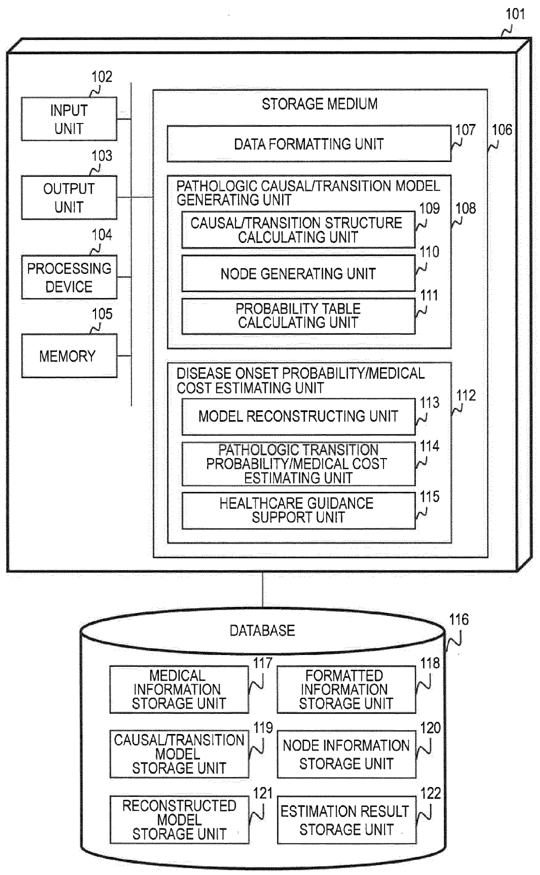


*Fig. 1*

EP 2 804 119 A2

**Description**

CLAIM OF PRIORITY

The present application claims priority from Japanese patent application JP 2013-104664 filed on May 17, 2013, the content of which is hereby incorporated by reference into this application.

BACKGROUND OF THE INVENTION

This invention relates to a data analysis technology, and more particularly, to a system for analyzing medical data, to thereby support a healthcare business.

A health insurance society operates an insurance business, a healthcare guidance system, for providing healthcare guidances for preventing lifestyle diseases, and preventing severity thereof from increasing in order to reduce medical cost. However, resources such as public health nurses employed for the healthcare guidance and a cost for the healthcare guidances are limited. Therefore, a system for supporting an operation of effective and efficient insurance business is desired.

As a method for supporting the operation of the insurance business, in Japanese Patent Application Laid-open No. 2012-128670 A, there is described a healthcare business support system for selecting people subject to a healthcare guidance based on healthcare cost information, health checkup information, and healthcare guidance information. The healthcare business support system includes a medical cost model generation unit for generating a medical cost model representing an estimated medical cost for each of severities and test values of an insured person to a health insurance, a test value improvement model generation unit for generating a test value improvement model representing an improvement amount for each of the severities and the test values, an estimated medical cost reduction effect calculation unit for calculating an estimated medical cost reduction amount by a healthcare guidance for each of the severities and the test values, and a subject person selection unit for selecting an insured person to the health insurance belonging to a severity and a test value high in estimated medical cost reduction amount as a healthcare guidance subject person.

SUMMARY OF THE INVENTION

It is necessary to select people subject to the healthcare guidance by priority in order to effectively and efficiently operate the insurance business within resources of a health insurance society. Moreover, a content of the healthcare guidance appropriate for each of the subject people needs to be selected.

When the medical cost is estimated according to Japanese Patent Application Laid-open No. 2012-128670 A, the future medical cost is estimated based on the current severity and test value. For example, a future severity of diabetes is estimated based on current severity and blood sugar level of diabetes, and an average medical cost corresponding to the severity is considered as an estimated medical cost.

However, a factor (blood sugar level for diabetes) effective for estimating the future medical cost and severity needs to be manually set as prior knowledge. Moreover, definition of the severity also needs to be manually set.

Various factors such as age, sex, other test values, a prescription state of medicines, and life style are considered in addition to blood sugar level as the factors effective for estimating the future medical cost, and more precise estimation can thus be carried out by considering the factors. However, it is difficult to manually list up the factors. Moreover, the factors need to be set based on the prior knowledge for each disease. Therefore, it is difficult to analyze all diseases.

The representative one of inventions disclosed in this application is outlined as follows. There is provided an analysis system comprising a processor configured to execute a program and a memory configured to store the program. The analysis system executes the program to analyze medical data. The analysis system is capable of making access to a database storing medical information including an injury and illness name of an insured person and a medical action performed on the insured person and health checkup information including a test value obtained by a health checkup on the insured person. The analysis system further comprises; a causation/transition structure calculating unit configured to control the processor to generate a graph structure including a node corresponding to an item defined by a plural of or the medical information or the health checkup information and a probability variable relating to the item, and a probabilistic dependency defined by one of a directed link or an undirected link between the nodes, and to store the generated graph structure in the database; a node generating unit configured to control the processor to generate an event space of the nodes based on the medical information and the health checkup information, and to store the generated event space in the database; a probability calculating unit configured to control the processor to calculate a conditional probability of the graph structure based on the medical information, the health checkup information and the event space, and to store the calculated conditional probability in the database; a state transition model reconstructing unit configured to control the processor to reconstruct a state transition model with a graph structure, an event space and a conditional probability including specified probability variables based on a state transition model constructed from the graph structure, the event space and the conditional probability, and to store the reconstructed state transition model in the database; a disease state transition estimating unit configured to control the processor to estimate a disease state transition probability based on the reconstructed state transition model; and a health guidance supporting unit configured to control the processor to select a subject for health guidance and a content of health guidance based on the estimated disease state transition probability.

According to one embodiment of this invention, a related future event can be accurately estimated based on various kinds of data. Objects, configurations, and effects other than those described above are readily apparent from the following description of embodiments.

BRIEF DESCRIPTION OF THE DRAWINGS

The present invention can be appreciated by the description which follows in conjunction with the following figures, wherein:

FIG. 1 is a block diagram illustrating a configuration of a medical data analysis system according to a first embodiment;
FIG. 2 is a block diagram illustrating a configuration of an analysis system according to a second embodiment;
FIGS. 3 to 5 are block diagrams illustrating other configurations of a medical data analysis system according to the first embodiment;
FIG. 6 is an explanatory diagram illustrating healthcare cost basic information according to the first embodiment;
FIG. 7 is an explanatory diagram illustrating health checkup information according to the first embodiment;
FIG. 8 is an explanatory diagram illustrating medical inquiry information according to the first embodiment;
FIG. 9 is an explanatory diagram illustrating injury and illness name information according to the first embodiment;
FIG. 10 is an explanatory diagram illustrating injury and illness name classification information according to the first embodiment;
FIG. 11 is an explanatory diagram illustrating clinical action information according to the first embodiment;
FIG. 12 is an explanatory diagram illustrating clinical action classification information according to the first embodiment;
FIG. 13 is an explanatory diagram illustrating medicine information according to the first embodiment;
FIG. 14 is an explanatory diagram illustrating medicine classification information according to the first embodiment;
FIG. 15 is an explanatory diagram illustrating an example of formatted information according to the first embodiment;
FIG. 16 is an explanatory diagram illustrating an example of formatted information according to the first embodiment;
FIGS. 17A to 17E are explanatory diagrams illustrating a model configured by two probability variables and the probability variables;
FIGS. 18A to 18C are explanatory diagrams illustrating a model configured by three probability variables and the probability variables;
FIGS. 19A and 19B are explanatory diagrams illustrating a model which is a Bayesian network;
FIGS. 20A to 20C are explanatory diagrams illustrating a process executed by a causation/transition structure calculating unit according to the first embodiment;
FIGS. 21A to 21C are explanatory diagrams illustrating node models of medical actions of this year and next year;
FIGS. 22A and 22B are explanatory diagrams illustrating models of a medical action, test value and lifestyle of this year and a medical action of next year;
FIGS. 23A to 23C are explanatory diagrams illustrating edges of causation/transition relations;
FIG. 24 is an explanatory diagram illustrating case numbers changed by a process of discretizing a node according to the first embodiment;
FIG. 25 is a flowchart for a process of discretizing a node according to the first embodiment;
FIGS. 26A and 26B are explanatory diagrams illustrating consolidation of nodes according to the first embodiment;
FIG. 27 is a flowchart for a process of consolidating nodes according to the first embodiment;
FIG. 28 is an explanatory diagram illustrating case numbers changed by a process of consolidating nodes according to the first embodiment;
FIG. 29 is a flowchart for a process of discretizing a node according to the first embodiment;
FIGS. 30A and 30B are explanatory diagrams illustrating examples of information stored in node information storage unit according to the first embodiment;
FIGS. 31A to 31C are explanatory diagrams illustrating consolidation of nodes according to the first embodiment;
FIG. 32 is an explanatory diagram illustrating an example of information stored in a causation/transition model storage unit according to the first embodiment;
FIGS. 33A to 33D are explanatory diagrams illustrating a process executed by a model reconstructing unit according to the first embodiment;
FIG. 34A is a flowchart of a process for a support function for a health insurance business operator according to the first embodiment;

FIG. 34B is a flowchart of a process for a support function for a person responsible for healthcare guidance and a subject person according to the first embodiment;

FIG. 35 is an explanatory diagram illustrating a simplified model directed to diabetes;

FIG. 36 is an explanatory diagram illustrating a model of a route for a directed edge;

Fig. 37A and 37B are explanatory diagrams illustrating data handled in the second embodiment; and

FIGS. 38A to 38C are explanatory diagrams illustrating models estimating medical cost of next year having edges illustrated in FIGS. 23A to 23C, respectively.

DETAILED DESCRIPTION OF THE PREFERRED EMBODIMENTS

[First Embodiment]

A first embodiment of this invention describes an example of a medical data analysis system that selects a person to be subjected to healthcare guidance, proposes a healthcare guidance approach, and estimates a healthcare guidance effect for prevention of disease onset and transition to a critical state thereof based on medical data (e.g., healthcare cost information, health checkup information, and medical inquiry information).

The healthcare cost information is information recording names of injury and illness when a health insurance insured person visits a medical institute for consultation, a prescribed medicine, a clinical action performed, and a medical costs (points). An example of the healthcare cost information is described later referring to FIG. 6. It should be noted that a prescribed medicine and a performed clinical action are generally referred to as "medical action".

Health checkup information stores a test value when an insured person has had a health checkup. An example of the health checkup information is described later referring to FIG. 7. Medical inquiry information stores the results of medical inquiry on lifestyle habit, an anamnestic history, a subjective symptom, etc. when an insured person has had a health checkup. An example of the health checkup information is described later referring to FIG. 8.

According to the first embodiment, a causal relation of a disease and disease state transition structure are modelized based on medical data. The first embodiment provides various functions such as selection of a person to be subjected to healthcare guidance, proposal of a healthcare guidance approach, and estimation of a healthcare guidance effect based on this model.

FIG. 1 is a block diagram illustrating the configuration of a medical data analysis system according to the first embodiment. FIGS. 3 to 5 are block diagrams illustrating other configurations of the medical data analysis system according to the first embodiment.

The medical data analysis apparatus 101 according to this embodiment includes an input unit 102, an output unit 103, a processing device 104, a memory 105, and a storage medium 106.

The input unit 102 is a human interface such as a mouse and a keyboard, and receives an input to the medical data analysis apparatus 101. The output unit 103 includes a display and a printer for outputting an arithmetic operation result by the medical data analysis system. The storage medium 106 is a storage apparatus for storing various programs for realizing medical data analysis processing by the medical data analysis system, an execution result of the medical data analysis processing, and the like, and is, for example, a non-volatile storage medium (such as a magnetic disk drive and non-volatile memory). The programs stored in the storage medium 106 are extended on the memory 105. The processing device 104 is an arithmetic operation apparatus for executing a program loaded on the memory 105, and is, for example, a CPU, a GPU, or the like. Processing and arithmetic operation described later are carried out by the processing device 104.

The medical data analysis system according to the first embodiment may be a computer system including a single computer, or a computer system including a server and client terminals. A data formatting unit 107, a disease causation/transition model generating unit 108, and a disease onset probability/medical cost estimating unit 112 of the medical data analysis apparatus 101 may be configured as separate apparatus as illustrated in FIGS. 3, 4, and 5. In this case, the apparatus illustrated in FIGS. 3 and 4 generate a model based on medical data. The apparatus illustrated in FIG. 5 provides a healthcare guidance supporting function to generate information for performing various kinds of healthcare guidance based on the generated model. A user uses the apparatus illustrated in FIG. 5. Because the apparatus illustrated in FIG. 5 used by the user need not hold medical data based on which the model has been generated, the apparatus is effective for concealment of personal information and prevention of leakage of personal information.

The medical data analysis system is a computer system configured on a single computer, or on a plurality of computers logically or physically constructed, and may operate on separate threads on the same computer or operate on virtual computers configured on a plurality of physical computer resources.

First, a description is given of medical data used in the first embodiment.

A medical information storage unit 117 stores the medical data input to the input unit 102. The medical data includes the healthcare cost information, the health checkup information, and the medical inquiry information. The healthcare cost information includes healthcare cost basic information, injury and illness name information, clinical action information, medicine information, injury and illness name classification information, clinical action classification information, and medicine classification information.

A description is now given of the healthcare cost information.

FIG. 6 is an explanatory diagram illustrating healthcare cost basic information 601.

The healthcare cost basic information 601 is information holding relationships between a healthcare cost and an insured person. The healthcare cost basic information 601 includes search numbers 602, insured person IDs 603, sex 604, ages 605, months and years of clinical action 606, and total points 607.

The search number 602 is an identifier for uniquely identifying a healthcare cost record. The insured person ID 603 is an identifier for uniquely identifying an insured person to the health insurance. The sex 604 is information representing a sex of the insured person. The age 605 is information representing an age of the insured person.

The month and year of clinical action 606 is a month and a year when the insured person visits a medical institute. The total point 607 is information representing a total point of one healthcare cost record. It should be noted that a medical cost (in yen) is calculated by multiplying the total point by "10". If a plurality of injury and illness names are registered to one search number in injury and illness name information 901 illustrated in FIG. 9, a total point of the medical action for the plurality of injuries/diseases is registered to the total point 607.

FIG. 9 is an explanatory diagram illustrating the injury and illness name information 901.

The injury and illness name information 901 includes search numbers 602, injury and illness codes 902, and injury and illness names 903.

The search number 602 is an identifier for uniquely identifying a healthcare cost record, and uses the same number as the search number (FIG. 6) of the healthcare cost basic information 601. The injury and illness code 902 is an injury and illness code described in the healthcare cost record. The injury and illness name 902 is an injury and illness name corresponding to the injury and illness code.

It should be noted that a plurality of injury and illness names can be described in one healthcare cost record. For example, the injury and illness names 903 of entries having "11" in the search number 602 are "diabetes" and "hypertension" in the injury and illness name information 901 illustrated in FIG. 9, and the injury and illness names, which are diabetes and hypertension, are described in the healthcare cost records having "11" in the search number.

FIG. 10 is an explanatory diagram illustrating injury and illness name classification information.

Injury and illness name classification information 1001 is information for associating injury and illness classification with injury and illness names belonging to the injury and illness classification with each other, and includes an injury and illness classification 1002, an injury and illness code 902, an injury and illness name 903, and a complication 1003.

The injury and illness classification 1002 is the classification to which an injury and illness in question belongs. The injury and illness code 902 is an injury and illness code described in the healthcare cost record, and uses the same numbers as used in the injury and illness code 902 of the injury and illness name information 901 illustrated in FIG. 9. The injury and illness name 903 is the name of an injury and illness corresponding to this injury and illness code, and uses the same names as used in the injury and illness name 903 of the injury and illness name information 901 illustrated in FIG. 9. The complication absence/presence 1003 indicates whether this injury and illness is the name of a complication

FIG. 11 is an explanatory diagram illustrating the clinical action information.

Clinical action information 1101 includes search numbers 602, clinical action codes 1102, clinical action names 1103, and clinical action points 1104.

The search number 602 is an identifier for uniquely identifying a healthcare cost record, and uses the same number as the search number (FIG. 6) of the healthcare cost basic information 601. The clinical action code 1102 is an identifier for identifying a clinical action described in the healthcare cost record. The clinical action name 1103 is a name of the clinical action described in the healthcare cost record. The clinical action point 1104 is a point relating to the clinical action.

In FIG. 11, for example, the clinical action names 1103 of "clinical action A" and "clinical action C" are described in healthcare cost records having "11" in the search number 602.

FIG. 12 is an explanatory diagram illustrating the clinical action classification information.

Clinical action classification information 1201 includes injury and illness classifications 1002, clinical action codes 1102, and clinical action names 1103.

The injury and illness classification 1002 uses the same classification as the injury and illness classification 1002 (FIG. 10) of the injury and illness name classification information 1001. The clinical action code 1102 is a clinical action code for identifying a clinical action practiced for the injury and illness in the injury and illness classification 1002, and uses the same code as the clinical action code 1102 (FIG. 11) of the clinical action information 1101. The clinical action name 1103 is a name of the clinical action corresponding to the clinical action code, and uses the same code as the clinical action name 1103 (FIG. 11) of the clinical action information 1101.

FIG. 13 is an explanatory diagram illustrating the medicine information.

Medicine information 1301 includes search numbers 602, medicine codes 1302, medicine names 1303, and medicine points 1304.

The search number 602 is an identifier for uniquely identifying a healthcare cost record, and uses the same number as the search number 602 (FIG. 6) of the healthcare cost basic information 601. The medicine code 1302 is a medicine code for identifying the medicine described in the healthcare cost record. The medicine name 1303 is a name of a medicine described in the healthcare cost record. The medicine point 1304 is an insurance point of the medicine.

In FIG. 13, for example, the medicine names of "oral antidiabetic A" and "hypertension oral medicine A" are described in healthcare cost records having "11" in the search number 602.

FIG. 14 is an explanatory diagram illustrating the medicine classification information.

Medicine classification information 1401 includes injury and illness classifications 1002, medicine codes 1302, and medicine names 1303.

The injury and illness classification 1002 uses the same classification as the injury and illness classification 1002 (FIG. 10) of the injury and illness name classification information 1001. The medicine code 1302 is a medicine code for identifying a medicine prescribed in the classification registered to the injury and illness classification 1002, and uses the same code as the medicine code 1302 (FIG. 13) of the medicine information 1301. The medicine name 1303 is a name of a medicine corresponding to the medicine code, and uses the same name as the medicine name 1301 (FIG. 13) of the medicine information 1303.

It should be noted that the clinical action information 1101 illustrated in FIG. 11 and the medicine information illustrated in FIG. 13 are generally referred to as medical action information. It should be noted that the clinical action classification information 1201 illustrated in FIG. 12 and the medicine classification information illustrated in FIG. 14 are generally referred to as medical action classification information.

A description is now given of the health checkup information.

FIG. 7 is an explanatory diagram illustrating the health checkup information.

Health checkup information 701 is information for managing health checkup information on a plurality of insured persons for a plurality of years, and includes insured person IDs 603, health checkup dates 702, and various test values (such as BMIs 703, abdominal circumferences 704, fasting blood sugars 705, systolic blood pressures 706, and neutral fats 707) in the health checkup.

The insured person ID 603 is an identifier of an insured person to the health insurance who has had the health checkup, and uses the same identifier as the insured person ID 603 (FIG. 6) of the healthcare cost basic information 601. The health checkup date 702 is the date of the health checkup. The BMI 703 to the neutral fat 707 are results of tests in the health checkup.

For example, if an insured person has not had a specific test, data in the health checkup information may be absent. For example, in FIG. 7, the items of a test received in 2004 of an insured person having an insured person ID "K0004" lack data for the systolic blood pressure 706.

A description is now given of the medical inquiry information.

FIG. 8 is an explanatory diagram illustrating the medical inquiry information.

Medical inquiry information 801 is information for managing medical inquiry information on a plurality of insured persons for a plurality of years, and includes insured person IDs 603, medical inquiry dates 802, and answers of the medical inquiry (such as smoking 803, drinking 804, and walking 805. The medical inquiry may include a life style, health history, a constitution such as allergy, and subjective symptoms.

The insured person ID 603 is an identifier of an insured person to the health insurance who has had the medical inquiry, and uses the same identifier as the insured person ID 603 (FIG. 6) of the healthcare cost basic information 601. The medical inquiry date 802 is the date of the medical inquiry. The smoking 803 to the walking 805 are results of the medical inquiry. The smoking 803 represents an average number of cigarettes smoked per day if the insured person has the smoking habit, and is "none" if the insured person does not smoke. The drinking 804 represents an average amount (unit: ml) of alcoholic beverages drunk per day if the insured person has the drinking habit, and is "none" if the insured person does not have the drinking habit. The walking 805 is an average period (unit: minute) of walking per day.

Detailed information such as the number of steps in the walking, the amount of drinking, and the number of smoked cigarettes may not be acquired from the medical inquiry. Not a specific amount of drinking, but a corresponding frequency out of frequencies classified in advance in a questionnaire may be responded. For example, if information only on absence/presence of habits of smoking and drinking are acquired, the frequency of drinking may be divided into a certain number of degrees (such as (1) none, (2) once to twice per week, (3) three times or more per week), and the frequency may be responded. In this case, the value in the medical inquiry information is the number without a quantitative meaning.

If an answer to a specific item is not received, data on the medical inquiry may be absent. For example, in FIG. 8, the items of a medical inquiry received in 2004 by an insured person having an insured person ID "K0003" lack data for the walking 805.

A description is now given of processing for a data formatting unit 107. The data formatting unit 107 sums/unifies and formats in a tabular form the healthcare cost information, the health checkup information, and the medical inquiry information for each of the insured persons and each period from the medical data stored in the medical information storage unit 117. In the following, a description is given while assuming one period is one year, but the one period may be another period such as half a year, two years, and three years.

FIG. 15 is an explanatory diagram illustrating an example of formatted information 1501. Referring to FIG. 15, a description is given of the processing by the data formatting unit 107.

The formatted information 1501 includes healthcare cost formatted information acquired by formatting the healthcare cost information in the year of 2004. Each row of the formatted information 1501 represents data summed for one insured person ID for one year.

An insured person ID 603, a sex 604, an age 605, and a total point 607 are the same as the insured person ID 603, the sex 604, the age 605, and the total point 607 (FIG. 6) of the healthcare cost basic information 601. A data year 1502 is a year of source data from which the formatted information is generated.

The injury and illness code 10 (1503) is the number of healthcare cost records having "10" in the injury and illness code out of the healthcare cost records for the insured person ID. The injury and illness code 20 (1504) is similarly the number of healthcare cost records having "20" in the injury and illness code out of the healthcare cost records for the insured person ID. The clinical action code 1000 (1505) is the number of healthcare cost records for which a clinical action having 1000 in the clinical action code has been performed out of the healthcare cost records for the insured person ID. The medicine code 110 (1506) is the number of healthcare cost record s for which a medicine having the medicine code of " 110" has been prescribed out of the healthcare cost records for the insured person ID.

A specific description is now given of the processing by the data formatting unit 107 for a case where the data in the year of 2004 are formatted.

First, one insured person ID is selected. The data formatting unit 107 acquires search numbers of healthcare cost records for the insured person ID having 2004 in the month and year of clinical action from the healthcare cost basic information 601. Then, the data formatting unit 107 refers to the injury and illness name information 901, and counts, for each injury and illness code, the number of healthcare cost records having the injury and illness code described thereon. As a result, the number of healthcare cost records for each of the injury and illness codes is acquired. Similarly, the data formatting unit 107 refers to the clinical action information 1101, counts the number of healthcare cost records for each of the clinical action codes, refers to the medicine information 1301, and counts the number of healthcare cost records for each of the medicine codes. As a result, a data row for the year of 2004 is generated for the selected insured person ID. This processing is carried out for all combinations of each of insured person IDs and each of the years subject to the analysis.

For example, search numbers "11", "12", and "13" can be acquired from the healthcare cost basic information 601 for the data of the insured person ID "K0001" in the first row in 2004 in the formatted information 1501 illustrated in FIG. 15. Referring to the injury and illness name information 901, the number of healthcare cost records having "10" in the injury and illness code is two corresponding to the search numbers "11" and "13" out of the three healthcare cost records. Thus, 2 is registered to the column of the injury and illness code 10 in the first row of the formatted information 1501.

The formatted information 1501 illustrated in FIG. 15 includes health checkup formatted information formatted from the health checkup information. Each row includes sums of data corresponding to a single insured person ID.

A value of each of the items is a value of the health checkup data of an insured person and a year respectively corresponding to the insured person ID 603 and the data year 1502. The health checkup data can be acquired from the health checkup information 701. If the health checkup information 701 includes a plurality of pieces of health checkup data corresponding to the same insured person ID and the same year, data on any one of dates of health checkup or an average of health checkup results of the plurality of times for the year may be used. If the data on one health checkup date is used, it is preferred to use data on a simultaneous health checkup day practiced at approximately the same time of every year. Moreover, data small in amount of deficiency may be selected. A numerical value defined in advance for representing the deficiency is used for the deficient data. In the example illustrated in FIG. 15, -1 is used. It should be noted that all values for an insured person who does not have the health checkup information are treated as the values of the deficient data.

The formatted information 1501 illustrated in FIG. 15 includes medical inquiry formatted information formatted from the medical inquiry information. Each row includes sums of data corresponding to a single insured person ID.

A value of each of the items is a value of the medical inquiry data of an insured person and a year respectively corresponding to the insured person ID 603 and the data year 1502. The medical inquiry data can be acquired from the medical inquiry information 801. If the medical inquiry information 801 includes a plurality of pieces of medical inquiry data corresponding to the same insured person ID and the same year, data on any one of the dates of medical inquiry or an average of medical inquiry results of the plurality of times for the year may be used. If the data on one health checkup date is used, it is preferred to use data on a simultaneous health checkup day practiced every year at approximately the same time of every year. Alternatively, data small in amount of deficiency may be selected. A numerical value defined in advance for representing the deficiency is used for the deficient data. In the example illustrated in FIG. 15, -1 is used. It should be noted that all values for an insured person who does not have the medical inquiry information are treated as the values of the deficient data.

As a result of the processing, healthcare cost formatted information, health checkup formatted information, and medical inquiry formatted information can be generated. The data only for the year of 2004 is illustrated in FIG. 15, but pieces of formatted data for other years are also generated.

On this occasion, when the healthcare cost formatted information is generated, similar items may be summarized, thereby unifying the plurality of items. For example, if functions of the oral antidiabetic A and functions of the oral antidiabetic B out of the items of the medicines are similar, the oral medicines A and B may be summarized, and may be treated as one item. On this occasion, a sum of the number of prescriptions of the oral antidiabetic A and the number of prescriptions of the oral antidiabetic B in the same year is used as a value of the item newly summarized. Criteria for determining whether items are similar or not are described below. Clinical action names belonging to the same injury and illness classification in the clinical action classification information 1201 are considered as similar items. Moreover, medicine names belonging to the same injury and illness classification in the medicine classification information 1401 are considered as similar items. Moreover, similar item information is manually generated in advance.

FIG. 16 is an explanatory diagram illustrating an example of the formatted information 1501 acquired by unifying the injury and illness code 10 and the injury and illness 20 of the healthcare cost formatted information. The value of an injury and illness code 1601 is a sum of the value of the injury and illness code 1503 and the value of the injury and illness code 1504 in FIG. 15, and is a sum of the number of healthcare cost records having "10" in the injury and illness code and the number of healthcare cost records having "20" in the injury and illness code.

The generated healthcare cost formatted information, health checkup formatted information, and medical inquiry formatted information shown in FIGS. 15 and 16 are stored in the formatted information storage unit 118 of the database 116. The formatted information 1501 is numerical data in a tabular form.

The value in the healthcare cost formatted information is acquired by summing the number of healthcare cost records, namely the number of prescriptions, but the value may be information representing whether the prescriptions exist or not. In other words, a case where the number of prescriptions is equal to or more than 1 (prescription exists) is summarized as 1, and a case where the number of prescriptions is 0 (no prescription) is set to 0, resulting in a binary representation. Moreover, the number of prescriptions may be considered to represent the severity, and the value of the healthcare cost formatted information may be a value representing a level as a result of classification of the number of prescription. For example, a case where the number of prescriptions is 0 is set to 0, a case where the number of prescriptions is 1 to 4 is set to 1, and a case where the number of prescriptions is equal to or more than 5 is set to 2, resulting in a representation as the three stages.

In the above-mentioned example, the healthcare cost formatted information, health checkup formatted information, and medical inquiry formatted information are summarized in a period of one year. However, the period may be set to a different period of two years, three years, or the like. In the following, a description is given of a case where the period for the summarizing is one year.

Next, the disease causation/transition model generating unit 108 is described.

The disease causation/transition model generating unit 108 includes a causation/transition structure calculating unit 109, a node generating unit 110, and a probability table calculating unit 111. The disease causation/transition model generating unit 108 generates a model representing a cause of a disease and disease state transition based on a graphical model by using formatted information stored in the formatted information storage unit 118.

Using the disease causation/transition model, an estimated value for a medical cost of a year (X+*n*) after a certain year (year X) can be calculated from personal health checkup data, medical inquiry data, and healthcare cost data of the certain year, to thereby estimate a disease onset probability. Further, an estimated value for a medical cost of the next year for a group in a specific state in year X (e.g., group whose blood sugar levels lie within a certain range) can be calculated, to thereby estimate the disease onset probability of a disease. Although the following describes estimation of a medical cost and the state of a disease of the next year (when *n*=1), the estimation may be made for a different period such as two years later or three years later.

At this time, generation of a model needs medical data acquired in years separated at least by n years. For n=3, for example, pieces of medical data acquired in years separated by three years, such as medical data of year 2004 and medical data of year 2007, are needed. The following description is given on the assumption that pieces of medical data acquired in years separated by *n* years are stored in the medical information storage unit 117, and formatted information generated from the medical data by the data formatting unit 107 is stored in the formatted information storage unit 118.

The disease causation/transition model generating unit 108 generates a model representing the cause of a disease and disease state transition by using the formatted information stored in the formatted information storage unit 118.

First, a graphical model is briefly described.

The graphical model is formed by nodes and edges. The node represents a probability variable, and the edge represents the dependency relation between nodes (between probability variables). The edges have two types: a directed link and an undirected link.

Now, two probability variables X1 and X2 are considered.

A structure 1701 illustrated in FIG. 17A shows the two probability variables X1 and X2 by circles, and a link directed to X2 from X1 by an arrow. The directed link indicates that the probability of the probability variable X2 taking each state depends on the state of the probability variable X1. In other words, the probability of each state of the probability variable X2 is given by a conditional probability P(X2 | X1). The probability variable X1 is referred to as a parent of the probability variable X2, and the probability variable X2 is referred to as a child of the probability variable X1.

Because the probability variable X1 does not have a parent node, the probability distribution of X1 is given by a prior probability P(X1). Therefore, the joint probability distribution of X1 and X2 is given by P(X1, X2)=P(X1)P(X2 | X1). X1 and X2 both take three values (states) of 1, 2, and 3. Expressing this probability distribution simply needs the probability distribution P(X1) and the probability distribution P(X2 | X1). The probability distribution P(X1) and the probability distribution P(X2 | X1) are expressed by a probability table 1702 and a probability table 1703, respectively, shown in FIGS. 17B and 17C. a*i* and a*ij* are probability values and are therefore real numbers of at least 0 and at most 1. Those probability values are expressed by ∑a*i*=1 (∑ being a sum for *i*), and ∑a*ij*=1 (∑ being a sum for *j*).

A structure 1704 illustrated in FIG. 17D shows an undirected link connected by two probability variables X1 and X2. The undirected link indicates that the probability variables are not independent. The probability variables X1 and X2 being independent indicate that the joint distribution P(X1, X2) of X1 and X2 can be broken down into P(X1, X2)=P(X1)P(X2). The probability table 1702 indicates that P(X1, X2)≠P(X1)P(X2). When it is unknown whether the probability variables X1 and X2 are independent, however, the case may be expressed as the structure 1704 in consideration of the possibility that the probability variables have a dependency. This probability distribution is expressed by a probability table 1705 shown in FIG. 17E. b*ij* is a real number of at least 0 and at most 1, and ∑b*ij*=1 (∑ being a sum for both *i* and *j*).

Accordingly, the dependency relation between probability variables can be expressed.

According to the first embodiment, a node (probability variable) is selected from items in formatted information of year X and items in formatted information of year X+*n*. For example, an injury and illness code 10 in year X, BMI in year X, smoking in year X, an injury and illness code 10 in year X+*n*, BMI in year X+*n*, smoking in year X+*n*, and so forth in FIG. 15 become nodes. Those nodes correspond to items in healthcare cost information, health checkup information, and medical inquiry information, or a unified item obtained by unifying a plurality of items in healthcare cost information.

The number of those items is, for example, several hundred to several thousand in consideration of the healthcare cost, health checkup, and medical inquiry when items in healthcare cost information are limited to those related to diabetes, and is several hundred thousand in consideration of all the items in healthcare cost information, all the items of health checkup, and all the items of medical inquiry. In other words, the number of nodes ranges from several hundred to as large as several hundred thousand.

The disease causation/transition model generating unit 108 uses formatted information generated from past healthcare cost information, health checkup information, and medical inquiry information to generate a model for estimating the disease onset probability and medical cost of a disease of an insured person n years after a certain year based on the healthcare cost information, health checkup information, and medical inquiry information for the insured person in the certain year. At this time, pieces of past formatted information of at least n years are needed. For n=3, for example, two years of past formatted information of year 2004 and year 2007 are used to generate a model for estimating the disease onset probability and medical cost of three years later. Assuming that the present year is 2008, and all or parts of the healthcare cost information, health checkup information, and medical inquiry information of an insured person are given, it is possible to estimate the disease onset probability and medical cost of year 2011 for the insured person.

FIG. 35 is an explanatory diagram illustrating a simplified model directed to diabetes.

The model illustrated in FIG. 35 is for estimating a oral antidiabetic prescribing state and an insulin prescribing state in year X+*n* based on a oral antidiabetic prescribing state, an insulin prescribing state, and a blood sugar level in year X. For example, the oral antidiabetic prescribing state in year X+*n* is given by a conditional probability of the oral antidiabetic prescribing state, the insulin prescribing state, and the blood sugar level in year X. The disease causation/transition model generating unit 108 automatically generates an edge and a conditional probability based on the formatted information. At the time of estimation, it is possible to set a current value to a node of year X and acquire a probability that the node will become an individual state in year X+*n* based on the model. Not all of the values of the nodes in year X need be given, but some of the values have only to be given.

The following description is given on the assumption that *n*=1.

The causation/transition structure calculating unit 109 forms an edge based on the dependency between those nodes. The node generating unit 110 generates space (event space) taken by the value of each node. The probability table calculating unit 111 calculates a conditional probability.

The causation/transition structure calculating unit 109 forms an edge (dependency relation) between those nodes (probability variables) from the data. The formation is described referring to a simple example.

Now, a model of determining whether a system including two sensors is normal or abnormal based on the statuses of the sensors is considered. Each of the probability variables, X1 and X2, which respectively indicate the statuses of the two sensors, takes two states. In addition, the system has a probability variable X3 which takes two statuses of normal and abnormal, respectively expressed by "0" and "1".

It is defined that when the sensors are in the status of " 1", it is likely that the system is abnormal. For example, X1 is for a temperature sensor and X1=1 is established when the temperature sensor indicates a temperature higher than a certain value, and X2 is for a sound sensor and X2=1 is established when the sound sensor detects a sound different from normal sounds. This implies that the case where the two sensors are effective in determining whether the system is normal or abnormal becomes a structure expressed by a structure 1801 in FIG. 18A. When the structure 1801 and a probability table 1802 are given, observation data obtained therefrom is expressed by, for example, a table 1803. Each row in the table 1803 represents a single piece of observation data. When each sensor shows an abnormal status, it is likely that the system is abnormal.

This example is described in comparison with the example illustrated in FIG. 18A. When pieces of observation data of X1, X2, and X3 are given as shown in the table 1803, the structure that matches the pieces of data is formed. According to this embodiment, the probability variables of X1, X2, X3, etc. are equivalent to items in formatted information, and one piece of observation data is equivalent to data of a single insured person ID.

When there are *N* probability variables, there are *M* models described above which are equal in number to the number of combinations (*M*) of two probability variables selected from among *N* probability variables in consideration of the presence/absence of an edge between probability variables. Accordingly, there are $2^M$ combinations of the presence/absence of an edge between nodes. In consideration of the direction of an edge, the types of models become greater. Therefore, it is not possible to check all the possibilities. To cope with this difficulty, there is a method of limiting expression of the model to a structure called Bayesian network to search for a structure suitable for expressing data.

The Bayesian network is a structure where every edge is a directed link, and there are no plurality of routes directed from a variable X1 to a variable X2 passing along directed links. For example, a structure 1901 illustrated in FIG. 19A is a Bayesian network, but a structure 1902 illustrated in FIG. 19B is not a Bayesian network.

Various methods of automatically learning the structures of a Bayesian network based on data are proposed. However, the use of any one of the methods has a difficulty in checking all the possibilities when the number of nodes becomes larger. When the scale of the system is large and different types and qualities of data are mixedly present as in the first embodiment, it is difficult to automatically learn an accurate network.

In this respect, the graphical model structure calculating unit 208 according to the first embodiment first defines the causation/transition relation between nodes as edges based on the features of the individual items of the healthcare cost, health checkup, and medical inquiry. Next, the graphical model structure calculating unit 208 calculates the dependency between nodes based on two dependencies, namely the quantitative dependency and co-occurrence dependency. Then, the graphical model structure calculating unit 208 deletes the edge between low-dependency nodes. In the graphical model according to the first embodiment, two types of edges, namely an edge expressing the pathologic cause and effect and an edge expressing a disease state transition, are taken into account.

The following describes the process executed by the causation/ transition structure calculating unit 109 referring to FIGS. 20A to 20C.

In causation/transition structure defining step 2001 in FIG. 20A, nodes are classified based on the features of the individual items of the healthcare cost, health checkup, and medical inquiry, and a causation/transition relation is defined as an edge between the classified nodes. It is an object of the model of the first embodiment to describe a cause and effect of a disease and estimate a disease state transition (disease onset). Accordingly, items are classified to items related to an injury and illness name, a medical action, a test value, a lifestyle habit, and basic information.

The injury and illness names are the items in the injury and illness codes 1503 and 1504 of healthcare cost formatted information, and the medical actions are the items in the clinical action code 1505 and the medicine code 1506 of the healthcare cost formatted information. The test value represents the items of a test value obtained by health checkup formatted information. The lifestyle habits are the items on the lifestyle habit and subjective symptom found by medical inquiry and obtained from the medical inquiry formatted information. The basic information represents the age and sex.

Nodes are classified based on the above-mentioned classifications of items. In other words, when nodes correspond to items in healthcare cost information, health checkup information, and medical inquiry information, the nodes are classified to the classifications to which the items belong, and when nodes correspond to a unified item obtained by unifying a plurality of items, the nodes are classified to the classification to which the unified item belongs. Through the above-mentioned processing, the nodes are classified into an injury and illness name, a medical action, a test value, a lifestyle habit, and basic information.

Referring to FIGS. 21A to 21C, the following describes some typical items for describing the process. In the diagrams, an injury and illness code 10 (injury and illness name), a medicine code 110 (medical action), a medicine code 120 (medical action), a blood sugar level (test value), etc. are treated as nodes (probability variables). What is presented within the parentheses represents classifications to which nodes belong. For the ease of understanding, in FIG. 21A, nodes such as the medicine code 120 (medical action) are replaced with words, such as "oral antidiabetic (medical action)", that help understand the nodes more easily.

It is an object of the model of the first embodiment to estimate the probability of a disease state transition (disease onset) and the medical cost in future and/or specify the cause of the disease state transition based on personal data of this year. To achieve the object, it is desired to estimate medical actions of the next year. The situations of this year's medical actions seem to be helpful information in the estimation of the next year's medical actions. Therefore, edges from the items of this year to items of the next year are generated between the nodes of this year's medical actions and the nodes of the next year's medical actions as seen from a structure 2101 illustrated in FIG. 21A. The structure 2101 illustrated in FIG. 21A shows that the probability of prescription for insulin next year depends on the probability of prescription for a oral antidiabetic, and the probability of prescription for insulin, and the probability of prescription for dialysis this year. In general, when the symptom of diabetes becomes serious, the medical actions are prescribed in the order of oral medicines, insulin, and dialysis.

The conditional probability for this model can be calculated by using data of two years of healthcare cost formatted information as shown in a table 2102 of FIG. 21B and a table 2106 of FIG. 21C. This makes it possible to calculate the probability that a person who has been given prescriptions only for oral medicines this year will be given a prescription for insulin or a prescription for dialysis next year. Such a dependency relation of the same classification items over a plurality of years is referred to as "transition", and other relations are referred to as "cause and effect".

It appears that the probability of transition varies depending on the test value and lifestyle habits of individual persons. For example, the probability that a person who is given a prescription for a oral antidiabetic this year will be given a prescription for insulin next year is expected to be higher for a person having a higher blood sugar level. Apparently, acquisition of more detailed personal information can provide a more rigorous probability of transition.

Because the probabilities of being given prescriptions for individual medical actions next year seem to depend on this year's test value, a directed link from this year's test value to the next year's medical action is defined. Likewise, a lifestyle habit seems to affect a next year's medical action, and hence a directed link from this year's lifestyle habit to the next year's medical action is defined. Those definitions are illustrated in a structure 2201 in FIG. 22A.

Further, a medical cost is calculated based on a medical action, and hence, when the medical cost is to be estimated, a directed link from the medical action of this year to the total points (medical cost) of the next year is defined. Further, in order to improve the accuracy of the medical cost, a directed link from this year's total points to the next year's total points is defined. Those definitions are illustrated in a structure 2202 in FIG. 22B.

The above-mentioned edges of the causation/transition relations are organized in a table 2301 shown in FIG. 23A. The table 2301 shows parents in rows and children in columns, and describes "transition" or "cause and effect" when there is an edge between a parent and a child, and shows a blank field when there is not such an edge. This model estimates the next year's medical actions from this year's test value, lifestyle habit, and medical actions, and estimates the next year's medical cost from this year's medical actions, the result of the estimation of the next year's medical actions, and this year's medical cost. FIG. 38A illustrates a node belonging to each classification by a single circle to schematically illustrate an edge between classifications.

Other definitions of the causation/transition relations are illustrated in FIGS. 23B and 23C.

A model expressed by a table 2302 illustrated in FIG. 23B estimates the next year's test value from this year's test value and this year's lifestyle habit, and estimates the next year's medical actions based on the next year's test value.

A model expressed by a table 2303 illustrated in FIG. 23C is a hybrid type combining the models expressed by the table 2301 and the table 2302, and estimates the next year's test value from this year's lifestyle habit and this year's test value. The model also estimates the next year's medical actions from this year's test value, this year's medical actions, and the next year's test value estimated.

FIGS. 38B and 38C schematically show edges between classifications corresponding to the causation/transition relations illustrated in FIGS. 23B and 23C, respectively.

The direction of an edge is described now. As illustrated in FIGS. 38A to 38C, an edge is defined to be directed from a lifestyle habit to a test value. This represents that the result of the lifestyle habit affects the test value. Likewise, an edge is defined to be directed from a lifestyle habit to a medical action, and from a lifestyle habit to total points. This represents that the result of the lifestyle habit affects the medical action and the total points. Further, an edge is defined to be directed from a test value to a medical action, and from a test value to total points. This represents that the result of the test value affects the medical action and the total points. Moreover, an edge is defined to be directed from a medical action to total points. This represents that the medical action affects the total points. Further, an edge is defined to be directed from a classification of year X to a classification of year X+*n*. Those combinations may be used in addition to the above-mentioned pair to define a structure.

Because the age and sex which are basic information are items that widely affect all the items in the causation/transition structure defining step 2001 in FIG. 20A, each of the age and the sex may be divided to generate different models. For example, a model may be generated with the age divided by every five years for male and female separately. When different models are not generated for different sex according to the age, but only a single model is generated, a directed link from this year's basic information to every item of the next year is defined. Any item other than the medical action, test value, lifestyle habit, and total points is excluded from a model, or a directed link from that item of this year to every item of the next year is defined.

Through the above-mentioned process, the direction and the presence or absence of an edge between nodes belonging to different classifications are defined. When the model illustrated in FIG. 23A is used, for example, a directed link is defined to be directed from a node belonging to the classification of test values of year X to a node belonging to the classification of a medical action of year X+*n*. Further, an edge from a node belonging to the classification of a lifestyle habit of year X to a node belonging to the classification of a lifestyle habit of year X+*n* is not defined.

The description of the process of the causation/transition structure defining step 2001 is completed. In the following, nodes with different periods are treated as nodes belonging to different classifications. In other words, the classification of test values of year X is treated as different from the classification of test values of year X+*n*.

Next, among the transition and causal edges between nodes (probability variables) belonging to different classifications defined in the causation/transition structure defining step 2001, the dependency between the probability variables is calculated, and an edge between low-dependency probability variables is eliminated.

In Inter-node dependency calculating step 2002, the dependency between nodes (probability variables) is calculated. At this time, individual nodes have values of different properties. For example, a test value, such as BMI or fasting blood sugar, is a sequential value whose scale varies from one value to another. The item "medical action" in healthcare cost formatted information is an integer value representing the number of prescriptions. Further, the answer number for a subjective symptom, for example, as a medical inquiry item is a value which does not have a quantitative meaning. Further, there is a missing value. Under such a circumstance, a method of comparing dependencies of variables of different properties with one another is needed.

The first embodiment shows an example of calculating the dependency between nodes using two references, namely, the quantitative dependency reference and the co-occurrence dependency reference. The quantitative dependency reference is a reference for calculating the similarity between values that have quantitative meanings, whereas the co-occurrence dependency reference is a reference for calculating the similarity between values that do not have quantitative meanings or between a value that has a quantitative meaning and a value that does not have a quantitative meaning.

First, a method of calculating the quantitative dependency is described. The dependency between two probability variables X1 and X2 is calculated. As observation data of X1 and X2, x1=(x11, x12, ..., x1*n*) and x2=(x21, x22, ..., x2*n*) are given, respectively. The quantitative dependency to be described below is example based on the coefficient of correlation when x1 and x2 are considered as vectors.

The correlation coefficient between the vectors x1 and x2 are defined as r(x1, x2). Because there is a missing value in x1 and x2, an element having a missing value in one of x1 and x2 is eliminated. When x1*i* is missing, for example, x2*i* is eliminated. Vectors with the missing dimension eliminated from x1 and x2 are given as v1=(v11, v12, ..., v1*m*) and v2=(v21, v22, ..., v2*m*).

Even if the value of the correlation coefficient r(v1, v2) may have substantially the same dependency, the value varies due to the difference in property between the values of v1 and v2. Therefore, the elements in v1 and v2 are independently rearranged at random first. The resultant vectors, w1 and w2, are expected not to have a dependency therebeween. Using the vectors, |r(v1, v2)| - |r(w1, w2)| is calculated. When |r(v1, v2)| < |r(w1, w2)|, it can be determined that there is not a quantitative dependency. Accordingly, the quantitative dependency in this case is set to 0, and quantitative dependencies in the other cases are set as |r(v1, v2)| - |r(w1, w2)|. This makes it possible to calculate the quantitative dependency compared with that in a random case (when there is not a dependency).

Here, the quantitative dependency is effective in comparing pieces of data having quantitative values. In an example 2005 illustrated in FIG. 20B, for example, there is an apparent quantitative correlation between x1 and x2. In an example 2006 illustrated in FIG. 20C, likewise, there is a quantitative correlation between x1 and x2, though smaller than that in the example 2005. Such a situation is possible in the case of the answer number for medical inquiry. Accordingly, the co-occurrence dependency is needed as an index to measure the degree of co-occurrence of values.

A method of calculating the co-occurrence dependency is described by way of example where dependency between two probability variables X1 and X2 is calculated.

As observation data of X1 and X2, x1=(x11, x12, ..., x1*n*) and x2=(x21, x22, ..., x2*n*) are given, respectively. The co-occurrence dependency to be described below is an example based on the entropies of x1 and x2.

First, as in the case of the quantitative dependency, vectors with missing values eliminated are set as v1 and v2. Next, the set of element pairs of the vectors v1 and v2 is set to S={(v1*i*, v2*i*)} (where *i* is an integer value of 1 to m). The number of the elements of S is *m*. For the elements of S, *p*=(p1, p2), the number of the elements of S equal to p is given by np. In addition, the number of different elements of S is given by L. Then, the entropy of a pair of v1 and v2 normalized with L is given by the following equation.

$$e(v1, v2)=\Sigma[(-np/m)\log(-np/m)]/L$$

where E is the sum of all the elements p of S. As in the case of the quantitative dependency, e(w1, w2) is calculated for randomized w1 and w2. e(v1, v2) is a positive value which becomes smaller as the degree of co-occurrence of v1 and v2 get larger. Accordingly, when the random and normalized e(v1, v2)/e(w1, w2) is larger than 1, it can be determined that v1 and v2 do not have a dependency relation. Further, e(v1, v2)/e(w1, w2) is a value equal to or larger than 0. Accordingly, the co-occurrence dependency when e(v1, v2)/e(w1, w2) is larger than 1 is set to 0, and the co-occurrence dependency in the other cases is set to 1-e(v1, v2)/e(w1, w2).

The quantitative dependency and the co-occurrence dependency which are defined in the above-mentioned manner are values equal to or larger than 0 and equal to or less than 1, and each dependency becomes greater as the value becomes larger. The dependencies are calculated for every pair of probability variables having edges defined in the causation/transition structure defining step 2001. In the following, the quantitative dependency is expressed as Q, and the co-occurrence dependency is expressed as C.

In Dependency calibration step 2003 in FIG. 20A, the values of the quantitative dependency Q and the co-occurrence dependency C are corrected. A correction function f is expressed by f(C). When correction is just scale conversion, f may be a linear function, or may be a more complex function. When f is a quadratic function, f can be given by the following equation.

$f(C)=\alpha C*C+\beta C+\gamma$

A method of determining parameters for f ($\alpha$, $\beta$, and $\gamma$ in the above-mentioned case) is described below. For the examples 2005 and 2006 respectively illustrated in FIGS. 20B and 20C, for example, it is desirable that the value of the quantitative dependency Q(x1, x2) of x1 and x2 of the example 2005 be close to the value of the co-occurrence dependency C(x1, x2) of the example 2006. Therefore, a plurality of combinations of the vectors x1 and x2 having various quantitative dependencies are prepared to calculate Q(x1, x2), and x2 whose values are changed to random values are prepared to calculate C(x1, x2). It is desirable that the value of Q(x1, x2) be close to the value of f(C(x1, x2), and hence the parameters of f are determined in such a way that the least square error of f(C(x1, x2) and Q(x1, x2) becomes smaller. In the examples 2005 and 2006, x2 in the example 2006 is obtained by converting the value of x2 in the example 2005 from 0 to 1, from 1 to 0, from 2 to 3, and from 3 to 2.

Accordingly, the dependency is defined by D=max{Q, f(C)}.

In Low-dependency inter-node edge deleting step 2004, of the edges determined in the causation/transition structure defining step 2001, an edge located between nodes having D smaller than a predetermined threshold is deleted.

Through the above-mentioned processing, the direction and the presence/absence of an edge between nodes belonging to different classifications are defined. In other words, for a node N1 and a node N2 belonging to different classifications, when an edge between the node N1 and the node N2 is defined in the causation/transition structure defining step 2001, and when the dependency between the node N1 and the node N2 is equal to or larger than a predetermined threshold, the edge defined in the causation/transition structure defining step 2001 is defined between the node N1 and the node N2. Otherwise, an edge is not defined between the node N1 and the node N2.

In Constraint structure learning step 2007, a final inter-node edge structure is determined. Three examples are described hereinbelow.

First, a first example is described. In the first example, only an edge between nodes that belong to different classifications defined by the processes up to Low-dependency inter-node edge deleting step 2004 and whose dependency is equal to or larger than the threshold is treated as an edge of a final disease causation/transition model. At this time, an edge between nodes that have the same period and belong to the same classification is not defined.

A second example is described. Between nodes that belong to different classifications, the method of the first example is used for definition, and the structure of an edge between nodes that belong to the same classification is learned using an existing structure learning method. The edge structure can be efficiently learned by limiting the edge structure that is constructed as a result of the learning to, for example, the structure of a Bayesian network. Accordingly, the presence or absence of an edge is defined between nodes belonging to the same classification, and the direction of the edge is further defined when the edge is directed. The structure of an edge between nodes belonging to different classifications has already been defined. The edge structure formed through the above-mentioned processing may not be a Bayesian network structure as a whole even when the structure between nodes belonging to the same classification is a Bayesian network structure.

A third example is described. The presence/absence and the direction of an edge between nodes that belong to different classifications are restricted based on edges that are defined by the processes up to Low-dependency inter-node edge deleting step 2004. There are four ways of making the definition: a definition when an edge is not present between nodes, a definition when there is an undirected edge, and two definitions when there is a directed edge (there are two definitions according to the direction of the edge). By way of contrast, when an edge is defined between nodes belonging to different classifications is defined through the processes up to Low-dependency inter-node edge deleting step 2004, restrictions are made to the two cases of the case where an edge is not present between nodes, or the case where an edge in the same direction is present. When an edge is not present through the processes up to Low-dependency inter-node edge deleting step 2004, a restriction that an edge is not present between nodes is made. Under this restriction, the edge structure of the entire nodes is learned using the existing structure learning method.

When the values of nodes need to be discretized using the existing structure learning method in the above-mentioned second and third examples, a method of performing discretization based on the ratio of the number of persons, which is described later in the description of a node generating unit 209 may be used.

The above completes the process of the causation/transition structure calculating unit 109. Through this process, a structure (edge) between nodes is determined.

To generate a probability table in the probability table calculating unit 111, the node generating unit 110 defines event spaces for nodes and consolidates the nodes. A node has a sequential value like a test value. When the value of the medical action in healthcare cost information is the number of the prescriptions, the estimation accuracy becomes low if the granularity of the number of prescriptions is small. Accordingly, discretization is preferred to be performed with an adequate granularity. When the individual numbers of prescriptions are treated separately, the number of events for each number of prescriptions becomes small, which may undesirably reduce the accuracy of the probability table or may make generation of the probability table difficult.

The event space may be manually defined beforehand. For example, the weight may be expressed in divided ranks of 5 kilograms, and the event spaces for the corresponding nodes may be set to {..., 50 to 54, 55 to 59, ...}. In this case, a collection of weight values from 50 kilograms to 54 kilograms is treated as a single event.

Further, another example of defining event space is described. The above-mentioned method requires defining of the event space for each node. For example, the height and the weight differ from each other in meaning and scale, and hence different divided levels are required to be defined. In the example to be described, the value is divided by the ratio of the number of persons. Therefore, event space can be defined by a uniform method that does not depend on nodes. Specifically, the scale is divided by every k%, and sub scales from the lower $p$% to ($p$+$k$)% are grouped into one. For example, provided that lower 5% of the weights of all the insured persons are put into a group of w1 kilograms or less, and lower 5% to 10% of the weights are put into a group of w1 kilograms to w2 kilograms, event spaces become {w1 or less, w1 to w2, ...}. For division by 5%, the number of states becomes 20.

Nodes may not be consolidated. When event spaces are given by the above-mentioned method and nodes are not consolidated, the process proceeds to the process of the probability table calculating unit 111. When the nodes are not consolidated, the number of cases for calculating a conditional probability become 0 in some circumstances. In this case, a process of estimating the conditional probability is needed, and this process is described later.

Next, an example in which the event space of a node is defined, and nodes are consolidated is described.

First, definition of the event space of a node is described. The event space of a node defines the state (value) a probability variable takes, and is generated by discretizing value space of a corresponding item.

Next, a discretization method is described. According to the first embodiment, discretization of a node is performed using two references. The first reference serves to achieve discretization in such a way that a sufficient number of cases for each state of the node after discretization are acquired. A sufficient number of cases are acquired when the discretization is rough, and hence a statistically reliable probability table can be generated. When the discretization is too rough, however, the dependency of the probability distribution of a child node with respect to the state of this node cannot be expressed adequately. In this respect, the second reference serves to achieve discretization in such a way that the expression of the dependency of the conditional probability distribution of a child node with respect to the state of this node after discretization is not lost.

FIG. 25 is a flowchart for the process of discretizing a node. The following describes an example of the process of discretizing a node without losing the expression of the dependency of probability of a child node while giving priority to the number of cases.

First, the necessity for discretization is described referring to an example 1701 illustrated in FIG. 17A.

Generation of a model requires generation of a probability table 1702 for X1 and a probability table 1703 for X2. For example, a22 in the probability table 1703 is the probability of X2=2 when X1=2, which needs a sufficient number of cases where X1=2 and X2=2. When the granularity of X1 is fine, the number of cases becomes few, and 0 depending on a situation. An insufficient number of cases raise a problem in that the probability value cannot be estimated or the reliability of the probability value is reduced. It is therefore necessary to perform discretization to provide an adequate granularity. For X1=1 and X1=2, when the probability distribution of X2, P(X2|X1=1), is substantially equal to the probability distribution of P(X2|X1=2), it is favorable to put the states X1=1 and X2=2 into a single state from the viewpoint of the number of cases and the amount of calculation.

First, a discretization method to obtain a sufficient number of cases for each state of the node after discretization is described.

Assume that X1 is a node of interest, and X2 is a child node thereof, which has already been adequately discretized. A case number 2401 illustrated in FIG. 24 represents the number of cases for each state of X1. The values in the case number 2401 which represent the individual states become larger from left to right. A test value or the like is a sequential value for its meaning, but can be expressed in the illustrated manner because a test value is expressed discretely if divided finely to the granularity of effective digits. When the BMI value is expressed in an accuracy of up to the second decimal place, for example, the leftmost field in 2401 represents the number of cases for 0.00 to 0.01, the second field from the left in 2401 represents the number of cases for 0.01 to 0.02, and so forth.

First, the leftmost state of X1 is selected in Minimum value state selecting step 2501. Here, a case number 2402 represents the number of cases for each state of X2 when X1 is in a state expressed by a minimum value. The values in the case number 2402 become larger from left to right similarly to the values in the case number 2401. Likewise, a case number 2403 represents the number of cases for each state of X2 when X1 takes a state larger than the minimum value by " 1".

In the following description, the currently selected state is S. The initial state of S is a state expressed by the minimum value of X1.

In Step 2502, the number of cases for each state of X2 conditioned to make X1=S is compared with a predetermined threshold. When the number of cases is smaller than the predetermined threshold, it is determined that the number of cases is insufficient, and the state is combined with a next state on the right (Step 2503). When there is no next state on the right, the state may be combined with a next state on the left. As two left states of X1 are put together, the number of cases becomes case numbers 2404 and 2405 illustrated in FIG. 24, thereby increasing the number of cases of the states of X1 put together. Thereafter, the combined state is set as S, and the process then returns to Step 2502. It should be noted that when all the states are combined into a single state, the process is terminated.

When the number of cases is sufficient, on the other hand, S is regarded as a completed state, and it is checked in Step 2504 if there is an uncompleted state (next state on the right) (Step 2504). When there is an uncompleted state, this state is set as S, and the process then returns to Step 2502. When there is not any uncompleted state, the process proceeds to Step 2505.

This process can carry out discretization in such a way that each state has a stable number of cases, as shown in a case number 2407 of FIG. 24.

Further, discretization is carried out so that the probability dependency of a child node with respect to a parent node is not lost. Specifically, a state "0" at the left end of the case number 2407 and an adjoining state "1" are selected (Step 2505), and when two probability distributions, P(X2|X1=0) and P(X2|X1=1), do not have a significant difference (NO in Step 2506), the state "0" and the state "1" are combined (Step 2507). This process is repeated until the probability distributions have a difference. Then, a next state in X1 on the right is targeted (YES in Step 2508), and states are combined by a similar method. Regarding a difference between P(X2|X1=0) and P(X2|X1=1), for example, when there are states a and b of X2 that make the difference between P(X2=a|X1=0) and P(X2=b|X1=0) equal to or larger than the predetermined threshold, it is determined that the probability distributions have a significant difference therebetween.

Specifically, two minimum states after combination, namely the left end state and an adjoining state in the example of the case number 2407, are selected in Step 2501. The selected states are set as S1 and S2, respectively. Next, the difference between P(X2|X1=S1) and P(X2|X1=S2) is determined in the above-mentioned manner in Step 2506. When the two probability distributions do not have a difference, the process proceeds to Step 2507. In Step 2507, the state S1 and the state S2 are combined, and the combined state is newly set as S1, after which the process proceeds to Step 2508. When the two probability distributions do not have a significant difference in Step 2506, the state S2 is newly set as S1, after which the process proceeds to Step 2508. In Step 2508, when there is a state next to S1 on the right, the state is set as S2, and the process proceeds to Step 2506. When there is not a state next to S1 on the right, the process is terminated.

The above-mentioned process can perform discretization of X1 with the child node X2 discretized.

Accordingly, discretization is performed on the nodes in order starting with a leaf node which does not have a child node. When there is a node of total points indicating a medical cost, the total-point node becomes a leaf node. The total-point node is discretized beforehand so that the granularity needed for estimation is provided. When there is not a total-point node, a node relating to a medical action becomes a leaf node. This discretization method is predetermined. When distinction is made based on the presence or absence of a prescription, for example, discretization is performed based on two states of 0 and at least 1. When a finer granularity is needed, discretization is performed based on three states of, for example, 0, 1 to 5, and at least 6.

The above process achieves recursive discretization from a leaf node to a root node (node which does not have a parent) in order.

Next, the node generating unit 209 consolidates nodes.

As mentioned above, discretization is carried out paying attention to only a relation with child nodes. However, when a certain node has two or more parents as shown in a structure 2601 illustrated in FIG. 26A, the probability table needs a case for the combination of the states of the probability variables of all of the parent nodes. In the case illustrated in FIG. 26A, for example, X4 has three parent nodes X1, X2, and X3. In this case, a case is needed for the combination of all the states of X1, X2, and X3. Accordingly, the parent nodes are consolidated, and the states of the consolidated nodes are combined, as illustrated in FIG. 26B.

FIG. 27 is a flowchart for a process of consolidating nodes.

Consolidation of some nodes and combination of the states thereof are considered. First, in Step 2701, it is determined whether there are cases equal to or larger in number than a predetermined number for the combination of all the states of a parent node. When the number of cases is sufficient, this process is terminated.

When the number of cases is insufficient, in Maximum dependency pair consolidating step 2702, the dependency of parent nodes is calculated in the same way as used in Inter-node dependency calculating step 2002 to select a pair of nodes having a maximum dependency. It is considered that similar nodes having high dependencies give similar influences on child nodes. In this respect, two nodes having high dependencies are consolidated into a new node. When the numbers of states of the original two nodes are n1 and n2, the number of states of the new node is n1 ×n2 which is the combination of the states of the two nodes. A structure 2602 shows a state where the node X2 and the node X3 are combined with the node X5 (FIG. 26B).

Next, the states of the consolidated nodes are combined in State combining step 2703. A process of combining the states of the consolidated nodes is described referring to FIGS. 28 and 29.

Similarly to FIG. 24, FIG. 28 illustrates the states of nodes. Unlike FIG. 24, however, FIG. 28 illustrates the states of X5, which is the combination of X2 and X3, two dimensionally as seen in a state 2801. The numbers of cases are arranged in such a way that the values of X2 become larger from left to right, and the values of X3 become larger from top to bottom.

First, a state at the upper left end is selected in Upper-left-end state selecting step 2901. In other words, the state is a combination that permits both X2 and X3 to take minimum values. In the following, a selected state is expressed by S. Initially, S is the state at the upper left end. Next, in Step 2902, the number of cases of each state of X4 with a condition of X5=S is checked to determine whether there are a sufficient number of cases. When there are a sufficient number of cases for each state of X4, the process for this state is terminated, and proceeds to Step 2905. In Step 2905, the state 2801 is searched downward from the top row and from the left end to the right end in each row for any uncompleted state, and the first uncompleted state found is set as S, after which the process returns to Step 2902.

When the number of cases for each state of X4 is insufficient, on the other hand, an optimal adjoining state to be combined is selected in Optimal-adjoining-state selecting step 2903. The adjoining states are uncompleted states adjoining a currently selected state above, below, to the left, and to the right. Among those four adjoining states, a state that less influences the conditional probability distribution of the child node X4 when combined is an optimal state to be combined. When there is not any uncompleted state, an optimal state is selected from adjoining completed states, and is combined with the currently selected state. The combined state is newly set as S, after which the process returns to Step 2902.

The following describes an example of a method of calculating an influence on the conditional probability distribution of the child node X4 in Step 2903. Assume that the currently selected state is a, adjoining states are b, and I(b)=max | P(X4=s | X5=a)-P(X4=s | X5=a) | where max is a function for selecting a state among the adjoining states b to all the states s of X4, which has the minimum value of I(b), as an optimal adjoining state.

Through the above-mentioned process, the states are combined two-dimensionally. A state 2804 schematically shows how the two-dimensional combination is achieved. The state 2804 is illustrated with lines between combined states deleted.

This process is recursively repeated from a leaf node toward a root node to complete consolidation of the nodes. This overcomes the problem such that combining the number of states of a parent node reduces the number of cases, which otherwise makes estimation difficult or reduces the estimation accuracy.

Next, the node generating unit 110 generates a causation/transition structure after consolidation of the nodes. The node generating unit 110 deletes nodes to be consolidated, and inserts the consolidated node which is newly generated by the consolidation. At this time, all the parent nodes of the nodes to be consolidated are set as parent nodes of the consolidated node. For example, as illustrated in FIGS. 31A to 31C, X2 and X3 are consolidated (FIG. 31B) from an original structure 3101 (FIG. 31A), yielding a structure 3103 which is newly inserted as X5 (FIG. 31C).

Finally, the node generating unit 110 stores information on this structure in a causation/transition model storage unit 119, and information on the consolidation of the nodes and the combination of the states in a node information storage unit 120. FIG. 32 illustrates an example of the information stored in the causation/transition model storage unit 119. A table 3201 represents the information on the structure 3103, and shows the parent nodes of each node. FIG. 30 illustrates an example of the information stored in the node information storage unit 120. State combination information 3001 indicates the combination of the states of each node, and is generated by the discretization process (FIG. 25). The state combination information 3001 shows that the values of BMI within a range from 18.01 to 20.00 form a single state. To-be-consolidated node information 3002 is a table showing consolidated nodes and nodes to be consolidated. The to-be-consolidated node information 3002 shows that a consolidated node 1 is the combination of a height node and a weight node. The state combination information of a consolidated node is the same as the state combination information 3001.

The probability table calculating unit 111 generates a conditional probability table with the structure that is generated by the node generating unit 110 and is stored in the causation/transition model storage unit 119. This is the calculation of P(X|X1, X2, ..., Xn) for each state of X, X1, ..., Xn where X, X1, ..., Xn are parent nodes of each node X.

The process is described with the structure 3103 illustrated in FIG. 31C taken as an example. Generation of P(X5|X6, X7) is considered. When X5, X6, and X7 each take two states of 0 and 1, it is necessary to calculate eight values obtained by changing a, b, and c to 0 and 1 in P(X5=a|X6=b, X7=c). For example, to calculate P(X5=0|X6=0, X7=0), first, all the cases that make X6=0 and X7=0 are extracted. The number of the extracted cases is set as p. Next, the number of cases in the p cases which make X5=0 is set as q. At this time, P(X5=0|X6=0, X7=0)=*q*/*p* is satisfied.

For example, X6 represents the blood sugar level in year X, X7 represents the presence or absence of a prescription for oral antidiabetic in year X, and X5 represents the presence or absence of a prescription for insulin preparation in year X+*n*; "1" represents the presence of a prescription. Assume that there is a prescription for oral antidiabetic in year X, there are p insured persons whose values of diabetes are expressed by S, and there are q insured persons, among the p insured persons, who have been given prescriptions for insulin preparation n years later. At this time, P(X5=1|X6=S, X7=1)=*q*/*p* is satisfied.

When there are no cases and thus the conditional probability cannot be calculated, a uniform distribution, for example, may substitute for the conditional probability. When *p*=0 is satisfied in the above-mentioned example, P(X5|X6=S, X7=1) cannot be calculated. Accordingly, the distribution of X5 is assumed to be uniform, and when X5 takes two values as in the above-mentioned example, P(X5=1|X6=S, X7=1)=1/2 and P(X5=0|X6=S, X7=1)=1/2 are set.

The probability table calculating unit 111 calculates this probability for all the nodes, and stores the generated probability table in the causation/transition model storage unit 119.

The above is the description of the process of the disease causation/transition model generating unit 108.

Next, the disease onset probability/medical cost estimating unit 112 is described. The disease onset probability/medical cost estimating unit 112 includes a model reconstructing unit 113, a disease state transition probability/medical cost estimating unit 114, and a healthcare guidance supporting unit 115.

The model reconstructing unit 113 reconstructs a model matching the user's intention from a causation/transition model stored in the causation/transition model storage unit 119 in response to a request from the healthcare guidance supporting unit 115. The reconstructed model is stored in a reconstructed model storage unit 121. The disease state transition probability/medical cost estimating unit 114 estimates disease onset probability and a medical cost by using the reconstructed model generated by the model reconstructing unit 113. The result of the estimation is stored in an estimation result storage unit 122.

First, the process of the model reconstructing unit 113 is described.

The model generated by the disease causation/transition model generating unit 108 is a large-scale model where a large number of nodes are related to one another. However, users are often interested in a part of this model. Therefore, the model reconstructing unit 113 provides a function of reconstructing only a model related to nodes necessary for a user. This not only can reduce the amount of calculation but also provides a model which is easy for a user to handle.

When a model is constructed from the beginning in response to a request from the user, a large amount of calculation is needed. However, the calculation cost for the reconstructing process is small. In this respect, information obtained from a huge amount of data can be used efficiently and effectively by a two-level configuration including the disease causation/transition model generating unit that generates an exquisite model, and the model reconstructing unit that reconstructs a compact model matching the purpose as employed in the first embodiment. When the system is configured differently by different apparatus as illustrated in FIGS. 3, 4, and 5, the user only needs to use the apparatus illustrated in FIG. 5. Once the apparatus illustrated in FIG. 3 or FIG. 4 generates a model, the apparatus of FIG. 5 used by the user need not hold medical data on which the generation of the model has been based, and hence the apparatus is effective for concealment of personal information and prevention of leakage of personal information.

The model reconstructing unit 113 reconstructs a model matching the user's intention in response to a request from the healthcare guidance supporting unit 115. In other words, when provided with a list of nodes to be included in a reconstructed model, the model reconstructing unit 113 constructs a model relating to the nodes. The node list includes nodes before consolidation. In other words, the nodes correspond to items in formatted information. For example, when a pathologic cause and effect and disease state transition which are related to diabetes are of interest, items, test values, and medical inquiry results that relate to associated medical actions are set up into a node list.

First, a description is given of the process of the model reconstructing unit 113 when the node generating unit 110 does not consolidate nodes, and a graphical model generated by the disease causation/transition model generating unit 108 is a directed graph.

As a list of nodes, N1, N2, ..., N*k* are selected. First, an edge structure is set in such a way that in the model generated by the disease causation/transition model generating unit 108, when there is a route moving through an edge directed from N*i* to N*j*, an edge directed from N*i* to N*j* is set, and when there is a route moving through an edge directed from N*j* to N*i*, an edge directed from N*j* to N*i* is set. Otherwise, an edge is not set. Next, a conditional probability that is defined by the edge structure is acquired by marginalization of nodes that are not included in the list.

For example, a model illustrated in FIG. 36 is the original model, and X1 and X3 are given as a list of nodes. There is a route for a directed edge from X1 to X3 in this example, and hence an edge directed from X1 to X3 is generated in a reconstructed model. The conditional probability P(X3|X1) is given by marginalization of X2 from the original model. In other words, a relationship of "P(X3=s3|X1=s1)=∑P(X3=s3|X2=s2)P(X2=s2|X3=s3)" is satisfied, where ∑ is the sum of all the states s2 of X2.

The process executed by the model reconstructing unit 113 when the node generating unit 110 consolidates nodes is described referring to an example illustrated in FIGS. 33A to 33D. A structure 3301 is a model which is generated by the disease causation/transition model generating unit 108, and is stored in the causation/transition model storage unit 119. X2 and X3 are consolidated to be X5. X3, X4, and X8 are given as a list of nodes. Given nodes and a node obtained by consolidating the given nodes, namely, X5, X4, and X8 in the structure 113, are selected from the model stored in the causation/transition model storage unit 119 as nodes of a reconstructed model.

Next, the model reconstructing unit 113 reconstructs a model including only X5, X4, and X8. At this time, when there is a route connecting the nodes selected as the nodes of the reconstructed model, a directed link is formed between the nodes even in the reconstructed model. A structure 3302 becomes a structure 3303.

Next, the conditional probability is calculated to complete the reconstructed model. An example of calculating P(X4|X5) is described instead of the description of the process. P(X4|X5) can be calculated by ∑P(X4|X1=s, X5), where ∑ is the sum of all the states of X1. In other cases, the conditional probability can also be acquired from the model stored in the causation/transition model storage unit 119.

The above-mentioned process reconstructs a model which is constructed from nodes selected as a list of nodes when there is consolidation of nodes, and from a node after consolidation when nodes are consolidated. The definition of an edge and the calculation of the conditional probability that follow the reconstruction of the model are the same as those when there is no node consolidation.

When all the nodes are specified as a list of nodes, the model reconstructing unit 113 need not perform model reconstruction, and hence the model generated by the disease causation/transition model generating unit 108 is used. Further, the model generated by the disease causation/transition model generating unit 108 may be used for the model that is used by the disease state transition probability/medical cost estimating unit 114 in the estimation, and the model reconstructing unit 113 may form only a network chart to be displayed on a display apparatus in the healthcare guidance supporting unit 115 into a reconstructed model. The network chart and the probability table in this case are based on the above-mentioned reconstructed model.

The disease state transition probability/medical cost estimating unit 114 estimates the disease onset probability of a disease and the medical cost thereof by using the model reconstructed by the model reconstructing unit 113, or the model generated by the disease causation/transition model generating unit 108 and stored in the causation/transition model storage unit 119.

This process is described using the structure 3302. In case of acquiring the probability of X5=s (e.g., X5 is an item relating to the number of prescriptions for insulin next year), the probability indicates a probability that the number of prescriptions for insulin becomes a number specified by s. The joint distribution of X1, X4, X5, X6, X7, and X8 is given by the following expression.

$$P(X1,\ X4,\ X5,\ X6,\ X7,\ X8)=P(X1)P(X6)P(X8)P(X7|X8)P(X5|X6,\ X7)P(X4|X1,\ X5)$$

P(X5=s) is given by the following expression, where ∑ is the sum of the states of all the probability variables except X5.

$$P(X5=s)=\sum P(X1,\ X4,\ X5,\ X6,\ X7,\ X8)$$

This calculation can be executed by using the probability table that is generated by the probability table calculating unit 111 and is stored in the causation/transition model storage unit 119. When there is a calculated probability variable other than X5 (e.g., when X1=t), the probability variable P(X5=s) is given by the following expression, where E is the sum of the states of all the probability variables except the observation node X1 and node X5 to be estimated.

$$P(X5=s)=\sum P(X1=t, X4, X5, X6, X7, X8)$$

This is equivalent to, for example, a case of estimating the next year's medical actions and medical cost while the state of an obtained test value of this year's health checkup as a node is fixed.

The above-mentioned processing can ensure estimation of the states of nodes equivalent to the next year's medical action and medical cost with this year's information obtained. When P(X) is acquired with the medical cost node being denoted by X, estimated probability values are acquired for the individual points of the medical cost. The next year's medical cost can be estimated as the expected value.

The above-mentioned expression calculates the sum of all the states, and hence it takes a considerable time for the calculation. Algorithms that efficiently acquire the sum have been proposed. Examples of the algorithms include the message passing algorithm and the junction tree algorithm. The disease state transition probability/medical cost estimating unit 114 may use those algorithms.

The healthcare guidance supporting unit 115 provides a function of supporting healthcare guidance for preventing future disease onset. The following describes two functions including a support function of supporting a health insurance business operator in preparing a healthcare guidance plan, and a function of supporting a person responsible for healthcare guidance or a subject person.

First, the support function of supporting a health insurance business operator in preparing a healthcare guidance plan is described. A health insurance business operator wants to select subject persons who get a high prevention effect brought by healthcare guidance by priority within the budget of healthcare guidance, and to perform guidance suitable for each subject person. There are a plurality of healthcare guidance services (healthcare guidance service 1, healthcare guidance service 2, etc.) that can be provided by a health insurance business operator. For example, the healthcare guidance service 1 is a guidance to mainly reduce the BMI value, and the healthcare guidance service 2 is a guidance to drop the cholesterol value.

The process for the support function for a health insurance business operator is described.

FIG. 34A is a flowchart of the process for the support function for a health insurance business operator.

First, in Subject disease setting step 3401, a target disease for the process is set. When the three major lifestyle-related diseases, namely, diabetes, lipid abnormality, and hypertension, are targets, for example, the model reconstructing unit 113 reconstructs a model by using items in medical actions, items in health checkup, and medical inquiry items corresponding to diabetes, lipid abnormality, and hypertension among items in healthcare cost formatted information. When all diseases are targets, the model generated by the disease causation/transition model generating unit 108 and stored in the causation/transition model storage unit 119 is used.

In next Healthcare guidance service setting step 3402, the types of healthcare guidance services and assumed effects of the individual healthcare guidance services are set. For example, the assumed effects of the healthcare guidance service 1 include a weight reduction of 5 kilograms.

In next Healthcare-guidance effect estimating step 3403, the effect of reducing the medical cost is estimated for all combinations of the healthcare guidance services and the subject candidates for the healthcare guidance. First, a description is given of how to calculate the effect of reducing the medical cost for the combination of the healthcare guidance service 1 and a healthcare-guidance subject candidate 1.

First, the next year's medical cost for the healthcare-guidance subject candidate 1 when the healthcare guidance service is not provided is estimated. Based on the healthcare cost, and the values of the health checkup and medical inquiry for the healthcare-guidance subject candidate 1 this year, the states of nodes corresponding to the items of this year are set, and the disease state transition probability/medical cost estimating unit 114 estimates the medical cost (C1). Next, values of test values that are improved by the healthcare guidance service are set in this year's values of the healthcare-guidance subject candidate 1, and the disease state transition probability/medical cost estimating unit 114 estimates the next year's medical cost (C2). C1 is the estimated medical cost when healthcare guidance is not provided and C2 is the estimated medical cost when healthcare guidance is provided, and hence, given that C3 is a cost needed for the healthcare guidance, the cost effectiveness for reducing the medical cost can be calculated from E=C1-C2-C3. This process is performed for all the combinations of the healthcare guidance services and the healthcare-guidance subject candidates to calculate the cost effectiveness E for reducing the medical cost.

Next, in Healthcare guidance contents designing step 3404, one of all the combinations of the healthcare guidance services and the healthcare-guidance subject candidates which provides a greatest cost effectiveness for reducing the medical cost is selected. Then, the selected healthcare-guidance subject candidate is treated as selected. Then, one of the combinations of the healthcare guidance services for unselected healthcare-guidance subject candidates and the unselected healthcare-guidance subject candidates which provides a greatest cost effectiveness for reducing the medical cost is selected. Then, the selected healthcare-guidance subject candidate is treated as selected. In this manner, the combinations of the healthcare guidance services for healthcare-guidance subject candidates and the healthcare-guidance subject candidates can be selected in descending order of the effect. Finally, the combinations that provide a large effect are selected within the range of the budget of healthcare guidance to set a healthcare-guidance subject candidate and the contents of healthcare guidance therefor.

In Effect estimating step 3405, the values of the cost effectiveness for reducing the medical cost of the combinations selected in Healthcare guidance contents designing step 3404 are summed up, and a value obtained by subtracting the healthcare guidance cost from the effect of reducing the medical cost is output as an effect.

Next, the process for the support function for a person responsible for healthcare guidance and a subject person is described.

FIG. 34B is a flowchart of the process for the support function for a person responsible for healthcare guidance and a subject person.

First, in Subject disease setting step 3401, a target disease for the process is set. When the three major lifestyle-related diseases, namely, diabetes, lipid abnormality, and hypertension, are targets, for example, the model reconstructing unit 113 reconstructs a model by using items in medical actions, items in health checkup, and medical inquiry items corresponding to diabetes, lipid abnormality, and hypertension among items in healthcare cost information. When all diseases are targets, the model generated by the disease causation/transition model generating unit 108 and stored in the causation/ transition model storage unit 119 is used.

Another example of the process of Subject disease setting step 3401 is described. A subject person or a person responsible for healthcare guidance selects a disease to be treated. In other words, an item corresponding to a certain medical action is selected. Then, the dependencies of this item with respect to all of the other items are calculated by methods similar to those of Steps 2002 and 2003. Then, those items which each have at least a certain level of dependency to the selected item are extracted, and the model reconstructed by the model reconstructing unit 113 based on a list of the selected item and the extracted items is used.

In disease onset probability calculating step 3406, with the states of all the nodes being not set, the disease state transition probability/medical cost estimating unit 114 estimates the next year's disease state transition probability and medical cost of each of the diseases. The disease state transition probability of each disease can be acquired as a probability that the next year's number of prescriptions for nodes relating the medical action corresponding to this disease is at least one. This can be considered as the average disease probability of the disease. Then, based on the healthcare cost, and the values of the health checkup and medical inquiry for the subject person of this year, the states of nodes corresponding to the items of this year are set, and the disease state transition probability/medical cost estimating unit 114 estimates the next year's disease state transition probability and medical cost for each disease. The disease probability of each disease at this point of time is the disease probability of the disease of the subject person. Accordingly, for each disease, the disease probability of the disease of the subject person is divided by the average disease probability of the disease to calculate how many times the average disease probability higher the risk of developing disease of the subject person is.

In High-risk disease presenting step 3407, a disease whose risk of developing disease is higher than the average disease probability by at least a predetermined threshold, and the risk of developing disease are presented. Accordingly, the subject person or the person responsible for healthcare guidance can know the risk of developing disease of the subject person.

In Item-to-be-improved presenting step 3408, a test value that has at least a certain level of dependency to the medical action node corresponding to the high-risk disease calculated in High-risk disease presenting step 3407 is presented. The dependency is calculated by methods similar to those of Steps 2002 and 2003 of FIG. 20A.

Next, in Step 3409 of allowing the user to input a target value (Target value user inputting step 3409), the user is prompted to input an improving target value (e.g., the target value of weight) for a test item presented in Item-to-be-improved presenting step 3408.

Finally, in Effect estimating step 3410, the test item input in Target value user inputting step 3409 is updated with the target value, the disease probability of the disease after the target is achieved is estimated by a method similar to the one used for Step 3406, and a change in the risk of developing disease is presented. Viewing a change in the risk of developing disease, the user can set the improving target, and make good use of the change in self-management.

The healthcare guidance supporting unit 115 may display a model to be used in analysis as a network chart. The healthcare guidance supporting unit 115 may also display the risk of developing disease in the vicinity of an edge. Accordingly, the user can easily grasp how the state of the disease changes and the factor that affects the change. This feature is effective at the time of preparing the contents of healthcare guidance and setting the target to be improved by the healthcare guidance.

According to the configuration of the first embodiment, the disease causation/transition model generating unit 108 constructs a graphical model constructed by nodes based on items in healthcare cost information, health checkup information, and medical inquiry information. Then, the model reconstructing unit 113 reconstructs a graphical model of an adequate scale matching the purpose. This configuration can ensure estimation using a compact model, and fast estimation. It is not necessary to handle a large-scale model including nodes which do not match the purpose, helping the user understand the structure of a model. This improves the readability and facilitates an analysis.

There is another approach to prepare a model from medical data purpose by purpose. However, this approach cannot cope with various purposes unless medical data is always held. This approach thus still has a problem from the viewpoint of concealment of personal information. When medical data is not held, a model is generated for each purpose based on an application previously assumed, and hence this approach can cope with only a specific purpose such as a specific disease. Further, the generation of a model from medical data involves a huge amount of calculation compared with reconstructing of a model, and is disadvantageous from the viewpoint of the amount of calculation. The apparatus of the configuration of the first embodiment can be separated as illustrated in FIGS. 3, 4, and 5, and hence once the apparatus of FIG. 3 or FIG. 4 is used to generate a model, the user can reconstruct a model and perform estimation by using only the apparatus of FIG. 5.

The causation/transition structure calculating unit 109 restricts the direction of an edge between nodes representing a medical cost, a medical action, a test value, and a lifestyle habit. This signifies that a lifestyle habit influences a test value, a test value influences a medical action, a medical action influences a medical cost, and those past states influence the states in future. Such restriction to the direction of an edge between nodes can reduce the amount of calculation for learning a structure, and can provide a model which is intuitively easy to understand.

The node generating unit 110 consolidate nodes to define event space from two viewpoints: to secure the number of cases at the time of generating a conditional probability table and to maintain the dependency of the probability distribution of a child node to a parent node. Accordingly, a statistically reliable conditional probability table can be generated, and the estimation accuracy can be increased. Further, the event space of a node (probability variable) can be made small, which is advantageous from the viewpoint of the amount of calculation.

The healthcare guidance supporting unit 115 estimates a future state of a disease and a future medical cost by using a reconstructed model. The model of the first embodiment considers various factors, and hence it is possible to achieve accurate estimation. In addition, with the presence of healthcare cost information, it is possible to cope with any target disease. Further, an intervention effect originating from healthcare guidance can be estimated by performing estimation with the current test value of an insured person replaced with a value representing an expected improvement originating from healthcare guidance.

Moreover, displaying a model which is used in those analyses in the form of a network chart enables a user to grasp the influence originating from a change in state of a disease, and is effective in preparing the contents of healthcare guidance and setting a target to be improved by the healthcare guidance. This model is a reconstructed model, and is a chart formed by nodes to which a user pays attention, and hence the user is likely to show an interest in the model.

According to the first embodiment, as described above, a future disease probability and a future medical cost can be estimated accurately based on medical data such as healthcare cost information, health checkup information, and medical inquiry information. Factors effective for estimation can be automatically selected based on data, thus ensuring estimation in view of multiple factors. Further, diseases included in healthcare cost information can be analyzed, and hence a healthcare-guidance subject and the contents of healthcare guidance which show high cost effectiveness can be selected for various diseases.

In addition, the analysis system configured to include the model generating function (causation/transition structure calculating unit 109) and the model reconstructing function (model reconstructing unit 113) can achieve fast estimation for various diseases with high concealment of personal information.

Specifically, with the analysis system configured to include the model generating function and the model reconstructing function, the model generating function generates an exquisite and large-scale model designed for all diseases (all items in the healthcare cost record, and health checkup items), and the model reconstructing function reconstructs a compact model matching the purpose. For example, the model generating function alone increases the scale of a model and increases the amount of calculation for estimation, and hence the model is difficult to use. Moreover, when only a specific disease is to be analyzed, a model including an irrelevant disease as well is difficult to use. A model designed for each purpose (e.g., diabetes, lipid abnormality, or hypertension) may be generated as another approach, but this approach requires a significant amount of calculation in order to construct a model, undesirably making it necessary to hold original data (healthcare cost information, and health checkup information).

In the first embodiment, the model generating function generates an exquisite and large-scale model designed for all diseases, and a model matching the purpose is reconstructed from the generated model. The amount of calculation for reconstructing a model is not huge, and hence a model can be reconstructed easily. Further, the reconstructed model is compact, and hence the calculation cost for estimation is small. As long as the model generated by the model generating function is held, original data is unnecessary, and hence confidential information (personal information) need not be held at the time of performing estimation. This ensures effective and efficient use of a large amount of data.

With items in the healthcare cost record and health checkup items serving as nodes, the nodes are generated from a graphical model including the states of the nodes as the values of the items and the probability dependencies between the nodes serving as edges. Accordingly, the state of a child node depends on the state of a parent node, and can be given by the conditional probability of the parent node.

The edges of the graphical model are characterized by the transition and a cause and effect. For example, the current lifestyle habit and the current test value have a causal relation therebetween, the current test value and the current clinical action have a causal relation therebetween, the current clinical action and a future clinical action have a transitional relation therebetween, and a future clinical action and a future medical cost have a transitional relation therebetween. Further, the current lifestyle habit and the current test value have a causal relation therebetween, the current test value and a future test value have a transitional relation therebetween, a future test value and a future clinical action have a causal relation therebetween, and a future clinical action and a future medical cost have a transitional relation therebetween. Further, the current medical cost and a future medical cost have a transitional relation therebetween.

For the above-mentioned model generating function to generate a large-scale model, the increase in the scale of a model may suffer an insufficient number of cases for defining the conditional probabilities of the parent nodes of the individual nodes. When a parent node is large, the probability distribution of the states of a child node is given by the combinations of the states of the parent node, requiring a sufficient number of cases for all the combinations of the states of the parent node. Accordingly, it is preferred that the resolution of the states of a parent node and the number of parent nodes be small. When the resolution of the states of a parent node and the number of parent nodes are small, however, the accuracy of a generated model drops. Therefore, the node generating unit 110 performs consolidation and discretization of parent nodes in such a way as to reduce the influence on the probability distribution of child nodes and provide a sufficient number of cases. This process is carried out in order from a leaf node toward a root node.

In addition, the model generating function generates a model for each of items that are always distinguished, i.e., for each age range and each sex of insured persons, making it possible to construct a highly usable model.

The healthcare guidance supporting unit selects a list of all or some of the probability variables of diabetes, hypertension, and lipid abnormality, and hence lifestyle-related diseases which are major causes to increase medical costs can be analyzed.

[Second Embodiment]

In a second embodiment of this invention, a graphical model is constructed based on tabular information formed from items and data entries. The following describes an example of an analysis system that estimates an unknown value of newly acquired data based on the constructed model.

FIG. 2 is a block diagram illustrating the configuration of an analysis system according to the second embodiment.

The analysis system according to the second embodiment includes a data analysis apparatus 201 and a database 214.

The data analysis apparatus 201 includes an input unit 202, an output unit 203, an processing device 204, a memory 205, and a storage medium 206. The configurations and functions of those components are identical to those of the input unit 102, the output unit 103, the processing device 104, the memory 105, and the storage medium 106 of the first embodiment, respectively.

First, data that is handled in the second embodiment is described. The data that is handled in the second embodiment is tabular data 3701 shown in FIG. 37A showing X1, X2, and so forth as item names with a single data entry shown in a single row. Each column has the values of data entries for a single item stored therein. This tabular data 3701 is stored in a tabular information storage unit 215.

In the second embodiment, a graphical model having items X1, X2, so forth as nodes (probability variables) is constructed. Nodes are hereinafter expressed by Xi representing item names. Each row corresponds to an insured person of the first embodiment, and respective items correspond to items in healthcare cost information, health checkup information, and medical inquiry information of the first embodiment.

A graphical model generating unit 207 constructs a graphical model having the items X1, X2, so forth as nodes.

A graphical model structure calculating unit 208 defines an edge between items. With prior knowledge given, the presence/absence of a node, and the type of a node may be restricted. Assuming that a structure is a Bayesian network, then there is an efficient algorithm that learns edge structures. At this time, the dependency between items may be calculated by a method similar to the one used by the causation/transition structure calculating unit 109, and when the dependency is equal to or less than a threshold, the structure may be learned with a restriction made to indicate absence of an edge. The generated edge structure is stored in a graphical model storage unit 216.

The node generating unit 209 performs a process similar to that of the node generating unit 110 according to the first embodiment. The generated node information is stored in a node information storage unit 217.

A probability table calculating unit 210 performs a process similar to that of the probability table calculating unit 111 according to the first embodiment. The generated probability table is stored in a graphical model storage unit 216.

An estimating unit 211 estimates an unknown value included in a new data entry made. When data 3702 shown in FIG. 37B is given, for example, the values of items X4 and X5 in the data 3702 are unknown. Accordingly, the values of the items X4 and X5 are estimated based on known values in the data 3702 and the model constructed by the graphical model generating unit 207. The known values correspond to the values of this year's healthcare cost information, health checkup information, and medical inquiry information according to the first embodiment, whereas the unknown values correspond to the values of the next year's healthcare cost information, health checkup information, and medical inquiry information according to the first embodiment.

A simple graphical model reconstructing unit 212 reconstructs a model constructed from a list of specified nodes. The simple graphical model reconstructing unit 212 performs a process similar to that of the model reconstructing unit 113 according to the first embodiment. The reconstructed model is stored in a reconstructed model storage unit 218.

A probability inferring unit 213 specifies a list of nodes needed depending on the purpose for the simple graphical model reconstructing unit 212 to reconstruct a model. Further, the probability inferring unit 213 estimates unknown values in data input from the input unit 202 by using the model reconstructed by the simple graphical model reconstructing unit 212. The results of the estimation are stored in an estimation result storage unit 219.

The analysis system according to the second embodiment may be a computer system including a single computer, or a computer system including a server and client terminals. The graphical model generating unit 207 and the estimating unit 211 of the analysis apparatus 201 may be configured as separate apparatus.

The analysis system is a computer system configured on a single computer, or a plurality of computers logically or physically constructed, and may operate on separate threads on the same computer or may operate on virtual computers configured on a plurality of physical computer resources.

Each server is provided with a program that is executed by the processing device 204 through a removable medium (CD-ROM, flash memory, or the like) or over a network, and is stored in a non-volatile storage apparatus which is a non-transitory storage medium. Therefore, it is preferred that the computer system include an interface that ensures reading from a removable medium.

As described above, according to the second embodiment, it is possible to accurately estimate an event which occurs in future based on various kinds of data other than medical data.

This invention is not limited to the above-described embodiments but includes various modifications. The above-described embodiments are explained in details for better understanding of this invention and are not limited to those including all the configurations described above. A part of the configuration of one embodiment may be replaced with that of another embodiment; the configuration of one embodiment may be incorporated to the configuration of another embodiment. A part of the configuration of each embodiment may be added, deleted, or replaced by that of a different configuration.

The above-described configurations, functions, processing modules, and processing means, for all or a part of them, may be implemented by hardware: for example, by designing an integrated circuit. The above-described configurations and functions may be implemented by software, which means that a processor interprets and executes programs providing the functions. The information of programs, tables, and files to implement the functions may be stored in a storage device such as a memory, a hard disk drive, or an SSD (a Solid State Drive), or a storage medium such as an IC card, or an SD card. The drawings shows control lines and information lines as considered necessary for explanation but do not show all control lines or information lines in the products. It can be considered that almost of all components are actually interconnected.

## Claims

**1.** An analysis system, comprising:

a processor configured to execute a program;
a memory configured to store the program,
the analysis system executing the program to analyze medical data,
the analysis system being capable of making access to a database storing medical information including an injury and illness name of an insured person and a medical action performed on the insured person and health checkup information including a test value obtained by a health checkup on the insured person;
a causation/transition structure calculating unit configured to control the processor to generate a graph structure including a node corresponding to an item defined by a plural of or the medical information or the health checkup information and a probability variable relating to the item, and a probabilistic dependency defined by one of a directed link or an undirected link between the nodes, and to store the generated graph structure in the database;
a node generating unit configured to control the processor to generate an event space of the nodes based on the medical information and the health checkup information, and to store the generated event space in the database;
a probability calculating unit configured to control the processor to calculate a conditional probability of the graph structure based on the medical information, the health checkup information and the event space, and to store the calculated conditional probability in the database;
a state transition model reconstructing unit configured to control the processor to reconstruct a state transition model with a graph structure, an event space and a conditional probability including specified probability variables based on a state transition model constructed from the graph structure, the event space and the conditional probability, and to store the reconstructed state transition model in the database;
a disease state transition estimating unit configured to control the processor to estimate a disease state transition probability based on the reconstructed state transition model; and
a health guidance supporting unit configured to control the processor to select a subject for health guidance and a content of health guidance based on the estimated disease state transition probability.

**2.** The analysis system according to claim 1, wherein
the database includes cost information on the medical action,
the node includes the cost information and the probability variable of the cost information,
the disease state transition estimating unit configured to control the processor to estimate medical cost in future based on the cost information and the reconstructed state transition model; and
the health guidance supporting unit configured to control the processor to select the subject for health guidance and the content of health guidance based on the estimated medical cost.

**3.** The analysis system according to claim 2, further comprising a data formatting unit configured to control the processor to acquire a frequency of the medical actions, a cost for the medical actions, and the health checkup information from the medical information and the health checkup information, to generate formatted information obtained by putting the acquired pieces of information together for each insured person and over every predetermined period, and to store the generated formatted information in the database.

**4.** The analysis system according to claim 3, wherein the causation/transition structure calculating unit is configured to generate a graph structure by using a frequency of the medical actions and a content of the health checkup information in the formatted information as probability variables.

**5.** The analysis system according to claim 1,
wherein the causation/transition structure calculating unit is configured to generate a graph structure including:

a directed link defined to be directed from a node corresponding to a test value in a first period toward a node corresponding to a medical action in a second period after a predetermined period from end of the first period; and
a directed link defined to be directed from a node corresponding to a medical action in the first period toward a node corresponding to the medical action in the second period.

**6.** The analysis system according to claim 1,
wherein the causation/transition structure calculating unit is configured to generate a graph structure including:

a directed link defined to be directed from a node corresponding to a test value in a first period toward a node corresponding to a test value in a second period after a predetermined period from end of the first period;
a directed link defined to be directed from a node corresponding to a lifestyle habit in the first period toward a node corresponding to the test value in the second period;
a directed link defined to be directed from a node corresponding to the test value in the second period toward a node corresponding to a medical action in the second period; and
a directed link defined to be directed from a node corresponding to a medical action in the first period toward a node corresponding to the medical action in the second period.

**7.** The analysis system according to claim 1, wherein the causation/transition structure calculating unit is configured to generate a graph structure including:

a directed link defined to be directed from a node corresponding to a medical cost in a first period toward a node corresponding to a medical cost in a second period after a predetermined period from end of the first period; and
a directed link defined to be directed from a node corresponding to a medical action in the second period toward a node corresponding to the medical cost in the second period.

**8.** The analysis system according to claim 3,

wherein the causation/transition structure calculating unit is configured to:

generate two vectors v1 and v2 having, as elements, values of events whose values are not missing in the two probability variables among events in the formatted information;
generate vectors w1 and w2 respectively obtained by rearranging the elements of the two vectors v1 and v2 independently;
acquire a similarity based on a quantitative relation of a value obtained by subtracting a correlation coefficient of the vector w1 and the vector w2 from a correlation coefficient of the vector v1 and the vector v2;
acquire a ratio of an entropy of a set of sets of the elements of the vector w1 and the elements of the vector w2 and an entropy of a set of sets of the elements of the vector v1 and the elements of the vector v2 as a co-occurrence-based similarity; and
calculate, as a probabilistic dependency between the two probability variables, a larger value of the two similarities whose values are calibrated with a transform function prepared so that the values of the acquired two similarities for the same vector become close to each other.

**9.** The analysis system according to claim 8, wherein the causation/transition structure calculating unit is configured to avoid defining any of an undirected link and a directed link between nodes whose probabilistic dependency is equal to or less than a predetermined reference.

**10.** The analysis system according to claim 8,
wherein the node generating unit is configured to:

consolidate nodes whose probabilistic dependency is equal to or larger than a predetermined reference to define the nodes as new nodes; and
reconstruct a state transition model with a graph structure of the newly defined nodes.

**11.** The analysis system according to claim 3, wherein the node generating unit is configured to join events having fewer cases for the nodes than a predetermined reference so that a number of cases which are coincident with each of sets of values of a parent node having a child node and values of the child node and which are present in the formatted information is equal to or larger than a predetermined reference, thereby defining an event space of the nodes.

**12.** The analysis system according to claim 1, wherein the node generating unit is configured to define an event space of a parent node having a child node by joining events which make a change in a conditional probability distribution of the child node equal to or less than a predetermined reference, thereby.

**13.** The analysis system according to claim 1,
wherein the health guidance supporting unit is configured to:

calculate an estimated effect by subtracting a second estimated medical cost obtained by replacing a test value of the insured person with a test value improved by a health guidance service, and a cost for the health guidance service from the estimated medical cost of the insured person; and
select a combination of an insured person and a health guidance service, which provides a higher calculated estimated effect than a predetermined reference, as a subject for health guidance and a content of health guidance.

**14.** The analysis system according to claim 1,
wherein the health guidance supporting unit is configured to:

calculate a first risk of developing disease based on a ratio of the disease state transition probability of the insured person and an average disease state transition probability;
calculate a second risk of developing disease by using a disease state transition probability obtained by replacing a test value of the insured person with the improvement target of a test value input by a user; and
compare the first risk of developing disease with the second risk of developing disease to generate data of a risk reducing effect obtained by improvement.

**15.** A health business support method for supporting health guidance by using a computer including a processor for executing a program and a memory for storing the program,

the computer being capable of making access to a database storing medical information including an injury and illness name of an insured person and a medical action performed on the insured person, health checkup information including a test value obtained by a health checkup on the insured person,
the health business support method including:

a causation/transition structure calculating step of generating, by the processor, a graph structure including a node corresponding to an item defined by a plural of or the medical information or the health checkup information and a probability variable relating to the item, and a probabilistic dependency defined by one of a directed link or an undirected link between the nodes, and to store the generated graph structure in the database;
a node generating step of generating, by the processor, an event space of the probability variables based on the medical information and the health checkup information, and storing the generated event space in the database;
a probability calculating step of calculating, by the processor, a conditional probability of the graph structure based on the medical information, the health checkup information and the event space, and storing the calculated conditional probability in the database;
a state transition model reconstructing step of reconstructing, by the processor, a state transition model with a graph structure, an event space and a conditional probability including specified probability variables based on the graph structure, the event space and the conditional probability, and storing the reconstructed state transition model in the database;
a disease state transition estimating step of estimating, by the processor, a disease state transition probability and a medical cost based on the reconstructed state transition model; and
a health guidance supporting step of selecting, by the processor, a subject for health guidance and a content of health guidance based on the estimated disease state transition probability.

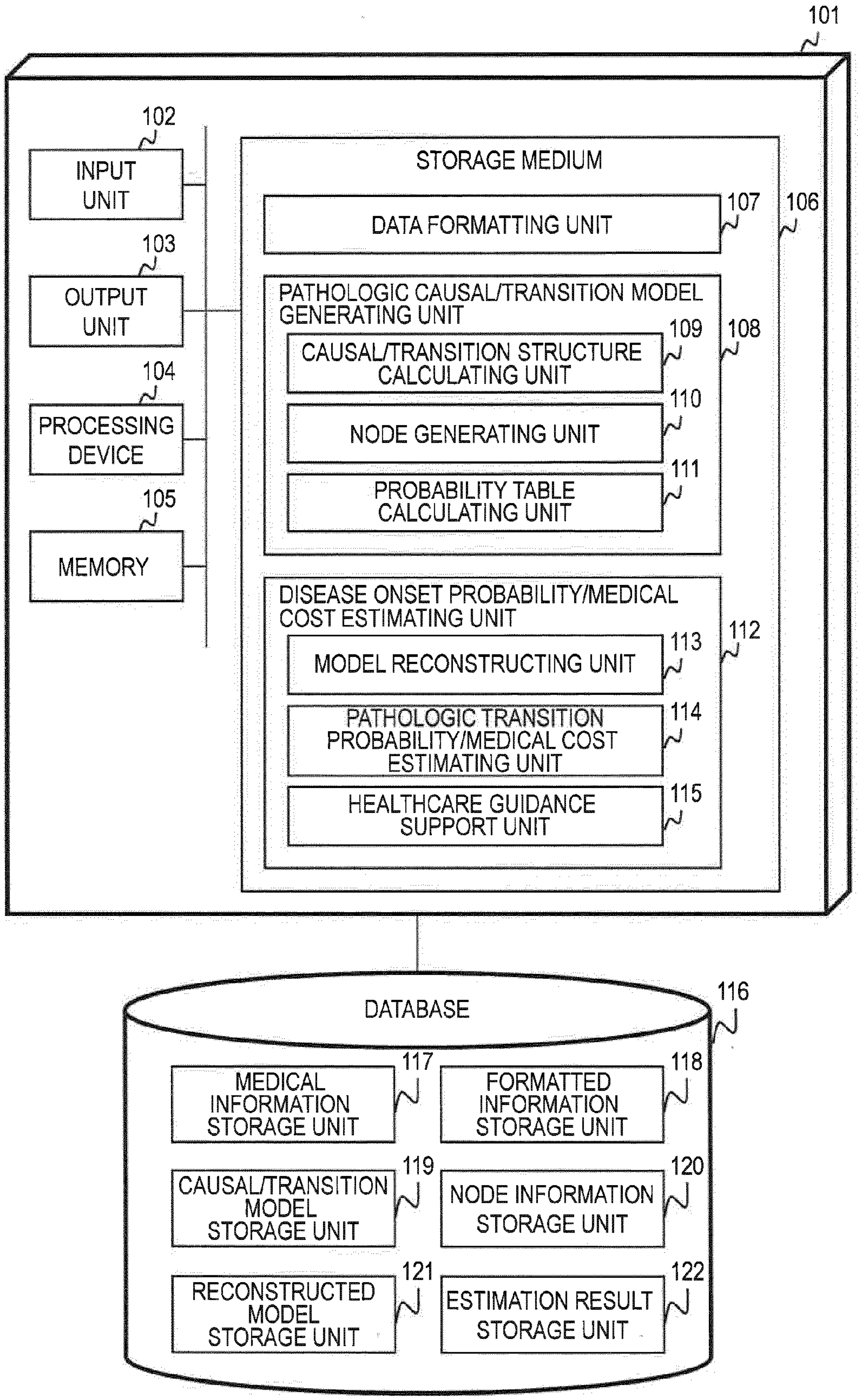
101
102
INPUT UNIT
103
OUTPUT UNIT
104
PROCESSING DEVICE
105
MEMORY
STORAGE MEDIUM
106
DATA FORMATTING UNIT
107
PATHOLOGIC CAUSAL/TRANSITION MODEL GENERATING UNIT
108
CAUSAL/TRANSITION STRUCTURE CALCULATING UNIT
109
NODE GENERATING UNIT
110
PROBABILITY TABLE CALCULATING UNIT
111
DISEASE ONSET PROBABILITY/MEDICAL COST ESTIMATING UNIT
112
MODEL RECONSTRUCTING UNIT
113
PATHOLOGIC TRANSITION PROBABILITY/MEDICAL COST ESTIMATING UNIT
114
HEALTHCARE GUIDANCE SUPPORT UNIT
115
DATABASE
116
MEDICAL INFORMATION STORAGE UNIT
117
FORMATTED INFORMATION STORAGE UNIT
118
CAUSAL/TRANSITION MODEL STORAGE UNIT
119
NODE INFORMATION STORAGE UNIT
120
RECONSTRUCTED MODEL STORAGE UNIT
121
ESTIMATION RESULT STORAGE UNIT
122

*Fig. 1*

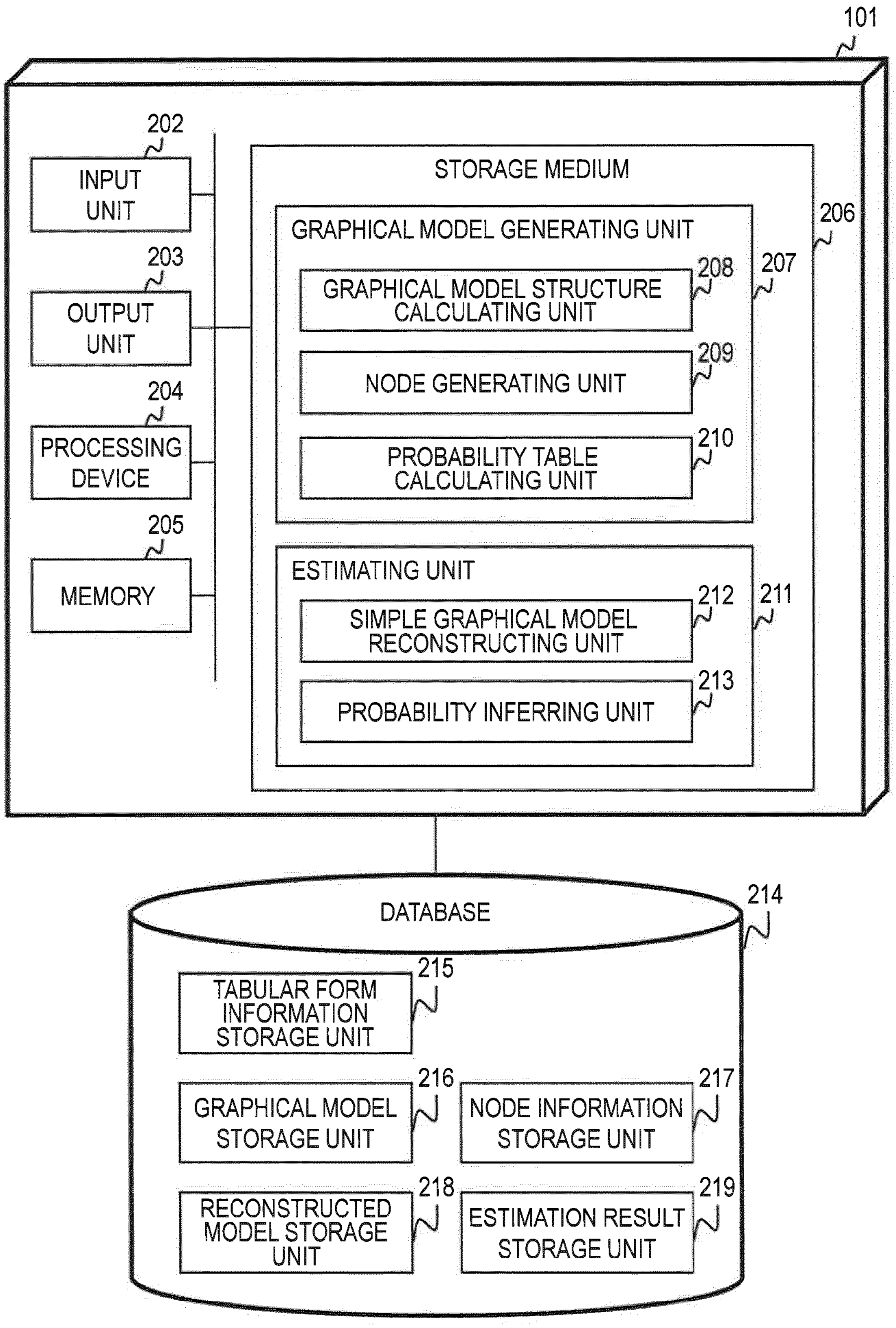
101
202
INPUT UNIT
203
OUTPUT UNIT
204
PROCESSING DEVICE
205
MEMORY
STORAGE MEDIUM
206
GRAPHICAL MODEL GENERATING UNIT
207
GRAPHICAL MODEL STRUCTURE CALCULATING UNIT
208
NODE GENERATING UNIT
209
PROBABILITY TABLE CALCULATING UNIT
210
ESTIMATING UNIT
211
SIMPLE GRAPHICAL MODEL RECONSTRUCTING UNIT
212
PROBABILITY INFERRING UNIT
213
DATABASE
214
TABULAR FORM INFORMATION STORAGE UNIT
215
GRAPHICAL MODEL STORAGE UNIT
216
NODE INFORMATION STORAGE UNIT
217
RECONSTRUCTED MODEL STORAGE UNIT
218
ESTIMATION RESULT STORAGE UNIT
219

*Fig. 2*

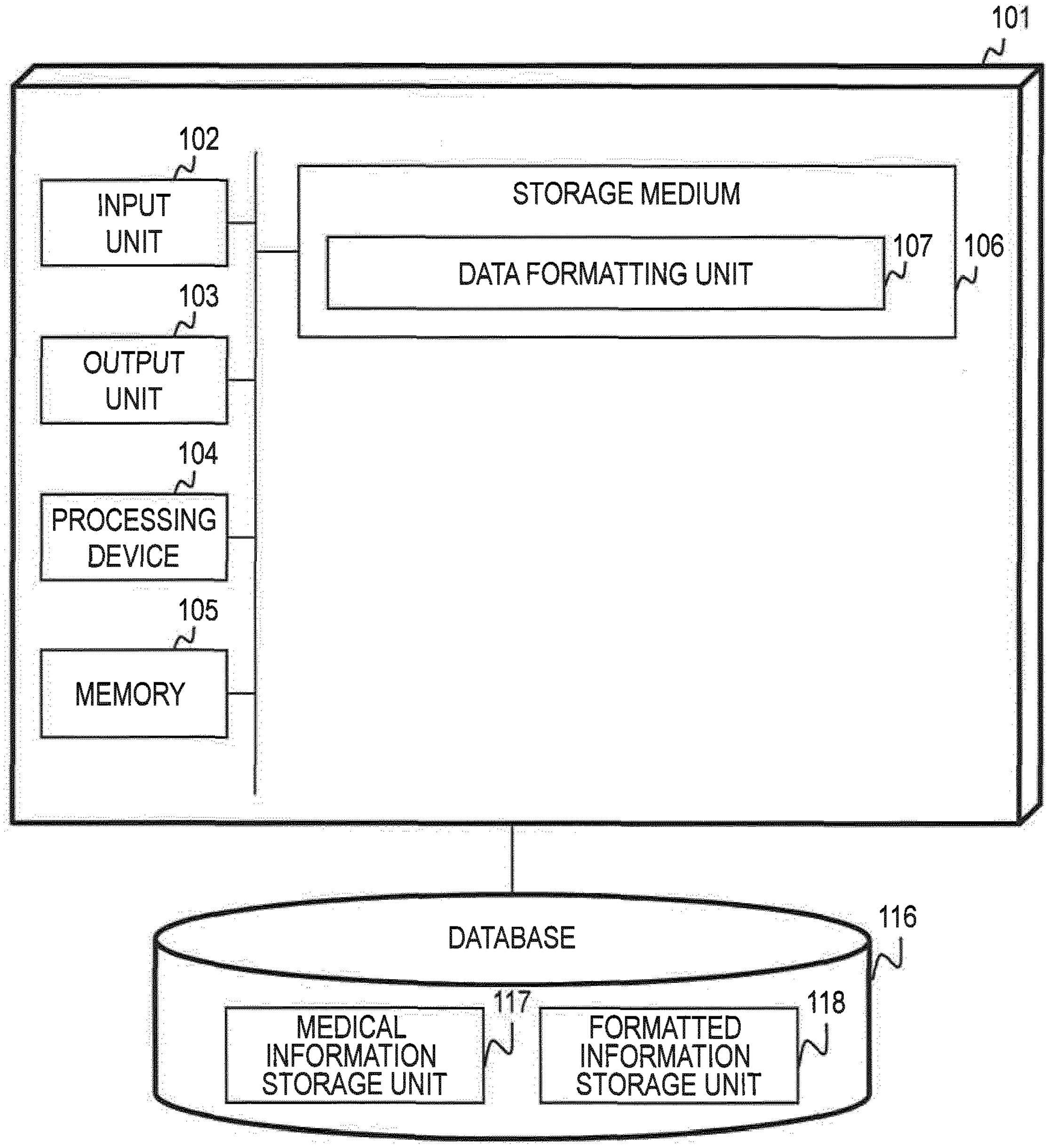
101
102
INPUT UNIT
103
OUTPUT UNIT
104
PROCESSING DEVICE
105
MEMORY
STORAGE MEDIUM
DATA FORMATTING UNIT
107
106
DATABASE
116
MEDICAL INFORMATION STORAGE UNIT
117
FORMATTED INFORMATION STORAGE UNIT
118

*Fig. 3*

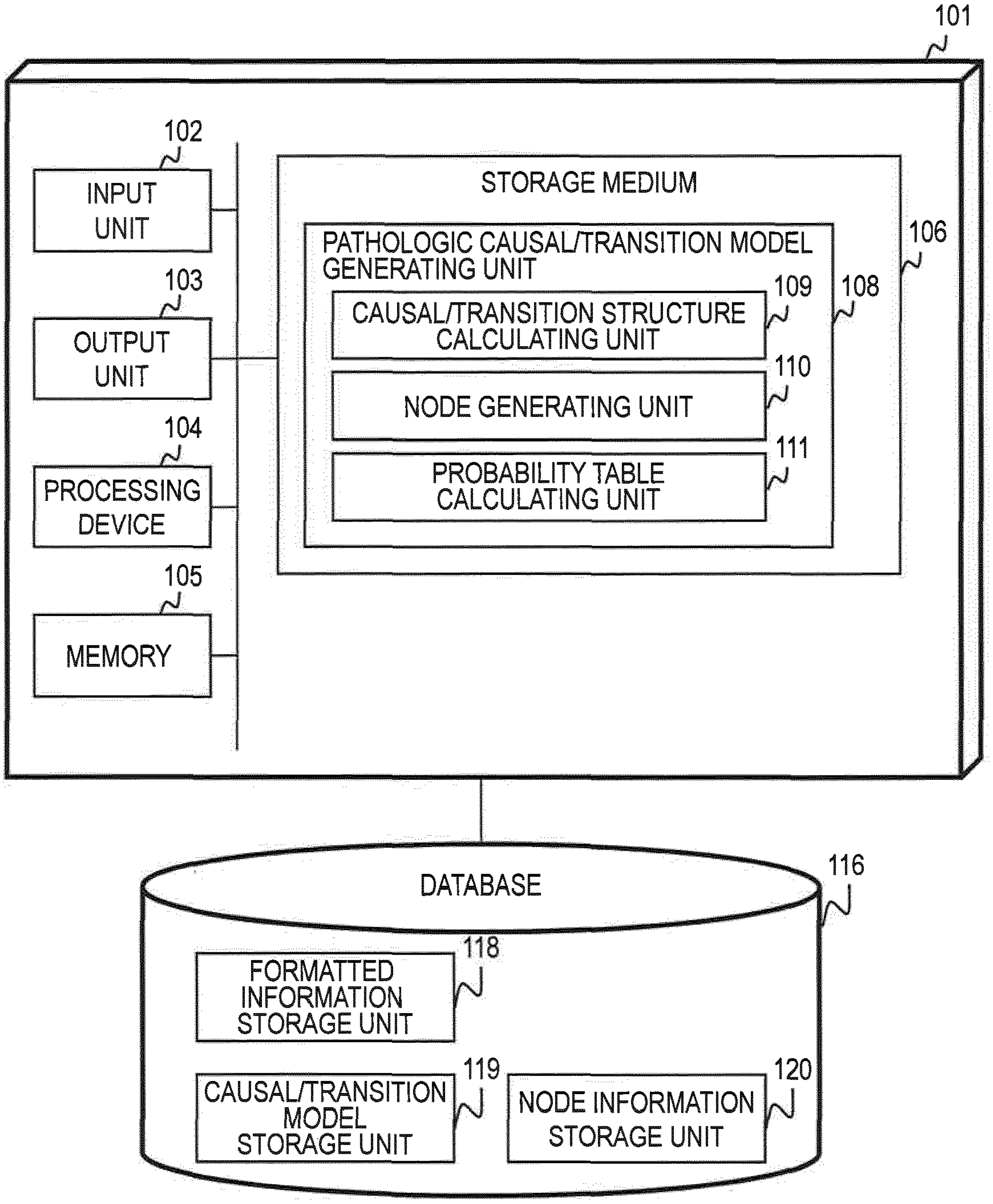
101
102
INPUT UNIT
103
OUTPUT UNIT
104
PROCESSING DEVICE
105
MEMORY
STORAGE MEDIUM
106
PATHOLOGIC CAUSAL/TRANSITION MODEL GENERATING UNIT
108
109
CAUSAL/TRANSITION STRUCTURE CALCULATING UNIT
110
NODE GENERATING UNIT
111
PROBABILITY TABLE CALCULATING UNIT
DATABASE
116
118
FORMATTED INFORMATION STORAGE UNIT
119
CAUSAL/TRANSITION MODEL STORAGE UNIT
120
NODE INFORMATION STORAGE UNIT

*Fig. 4*

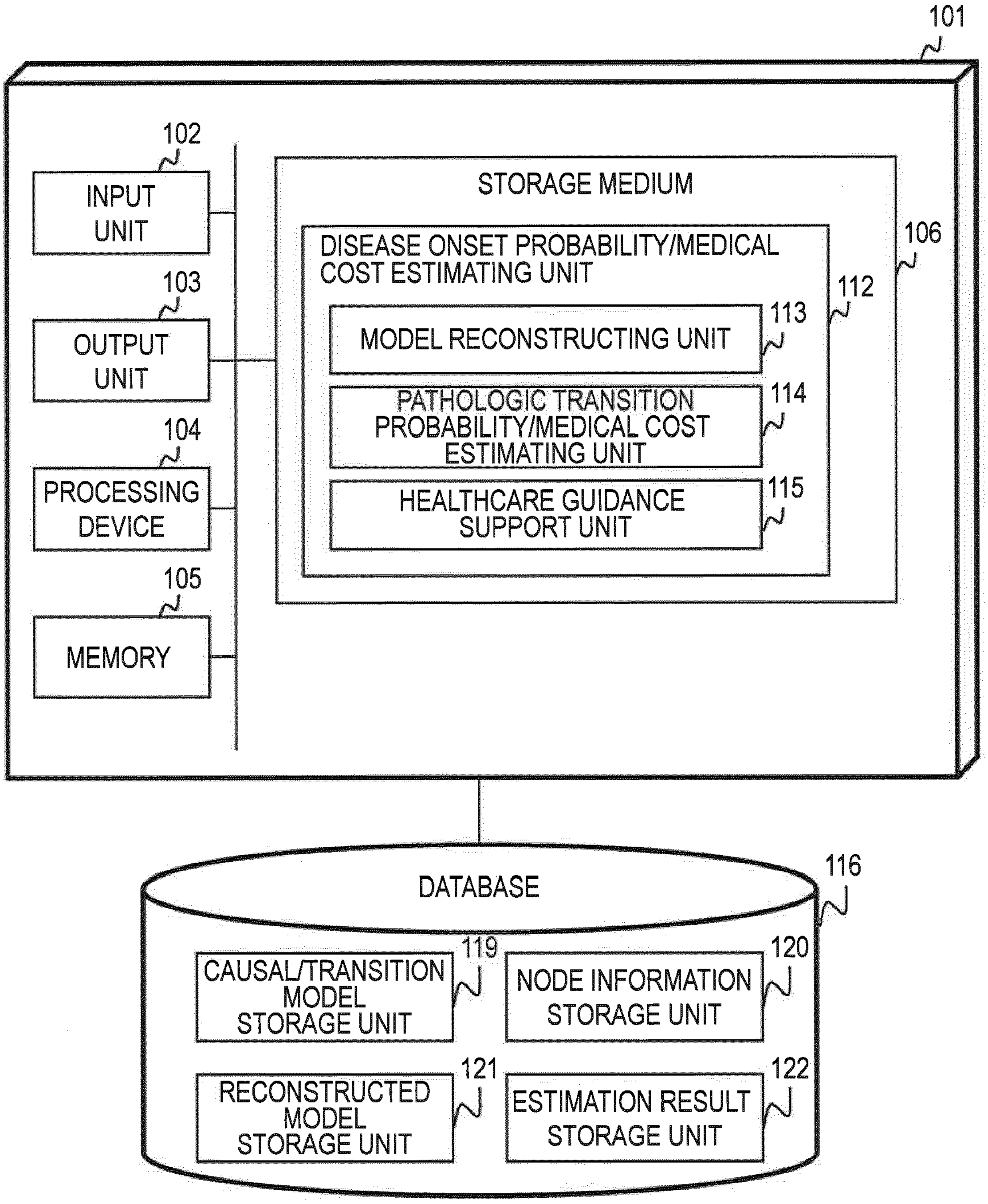
101
102
INPUT UNIT
103
OUTPUT UNIT
104
PROCESSING DEVICE
105
MEMORY
STORAGE MEDIUM
106
DISEASE ONSET PROBABILITY/MEDICAL COST ESTIMATING UNIT
112
MODEL RECONSTRUCTING UNIT
113
PATHOLOGIC TRANSITION PROBABILITY/MEDICAL COST ESTIMATING UNIT
114
HEALTHCARE GUIDANCE SUPPORT UNIT
115
DATABASE
116
CAUSAL/TRANSITION MODEL STORAGE UNIT
119
NODE INFORMATION STORAGE UNIT
120
RECONSTRUCTED MODEL STORAGE UNIT
121
ESTIMATION RESULT STORAGE UNIT
122

Fig. 5

CLAIMS DATA BASIC INFORMATION

601

| SEARCH NUMBER (602) | HEALTH INSURANCE INSURED PERSON ID (603) | SEX (604) | AGE (605) | YEAR/MONTH OF CLINICAL ACTION (606) | TOTAL POINT (607) | ... |
|---|---|---|---|---|---|---|
| 11 | K0001 | MALE | 40 | 2004/06 | GT11 | |
| 12 | K0001 | MALE | 40 | 2004/07 | GT12 | |
| 13 | K0001 | MALE | 40 | 2004/08 | GT13 | |
| 21 | K0002 | MALE | 45 | 2004/04 | GT21 | |
| 22 | K0002 | MALE | 45 | 2004/09 | GT22 | |
| 31 | K0003 | MALE | 50 | 2004/06 | GT31 | |
| 32 | K0003 | MALE | 50 | 2004/12 | GT32 | |
| 41 | K0004 | FEMALE | 50 | 2005/01 | GT41 | |
| ... | | | | | | |

*Fig. 6*

HEALTH CHECKUP INFORMATION

701

| HEALTH INSURANCE INSURED PERSON ID (603) | DATE OF HEALTH CHECKUP (702) | BMI (703) | ABDOMINAL CIRCUM-FERENCE (704) | FASTING BLOOD SUGAR (705) | SYSTOLIC BLOOD PRESSURE (706) | NEUTRAL FAT (707) | ... |
|---|---|---|---|---|---|---|---|
| K0001 | 2004/05/09 | 25 | 87 | 105 | 130 | 150 | |
| K0001 | 2005/05/15 | 24 | 84 | 100 | 125 | 130 | |
| K0002 | 2004/05/13 | 22 | 70 | 110 | 119 | 90 | |
| K0002 | 2005/05/20 | 21 | 70 | 108 | 119 | 90 | |
| K0003 | 2004/06/05 | 25 | 87 | 110 | 120 | 150 | |
| K0003 | 2005/06/10 | 23 | 87 | 111 | 122 | 155 | |
| K0004 | 2004/02/10 | 20 | 92 | 90 | - | 140 | |
| K0004 | 2005/02/20 | 20 | 90 | 90 | 120 | 130 | |
| ... | | | | | | | |

*Fig. 7*

MEDICAL INQUIRY INFORMATION

801

| HEALTH INSURANCE INSURED PERSON ID (603) | DATE OF MEDICAL INQUIRY (802) | SMOKING (803) | DRINKING (804) | WALKING (805) | ... |
|---|---|---|---|---|---|
| K0001 | 2004/05/09 | NONE | 100 | 20 | |
| K0001 | 2005/05/15 | NONE | NONE | 40 | |
| K0002 | 2004/05/13 | 10 | 200 | 10 | |
| K0002 | 2005/05/20 | 7 | NONE | 60 | |
| K0003 | 2004/06/05 | NONE | 50 | - | |
| K0003 | 2005/06/10 | NONE | SLIGHT | 20 | |
| K0004 | 2004/02/10 | 15 | NONE | 30 | |
| K0004 | 2005/02/20 | 3 | NONE | 10 | |
| ... | | | | | |

*Fig. 8*

INJURY AND ILLNESS NAME INFORMATION

602 902 903 901

| SEARCH NUMBER | INJURY AND ILLNESS CODE | INJURY AND ILLNESS NAME | ... |
|---|---|---|---|
| 11 | 10 | DIABETES | |
| 11 | 20 | HYPERTENSION | |
| 12 | 30 | BONE FRACTURE | |
| 13 | 10 | DIABETES | |
| 13 | 20 | HYPERTENSION | |
| 13 | 40 | GOUT | |
| 21 | 10 | DIABETES | |
| 21 | 11 | DIABETIC NEPHROPATHY | |
| 22 | 10 | DIABETES | |
| 22 | 11 | DIABETIC NEPHROPATHY | |
| 22 | 20 | HYPERTENSION | |
| 31 | 10 | DIABETES | |
| 32 | 10 | DIABETES | |
| 41 | 50 | INFLUENZA | |
| ... | | | |

*Fig. 9*

INJURY AND ILLNESS NAME CLASSIFICATION INFORMATION

1002 902 903 1003 1001

| INJURY AND ILLNESS CLASSIFICATION | INJURY AND DISEASE CODE | INJURY AND ILLNESS NAME | COMPLICATION |
|---|---|---|---|
| DIABETES | 10 | DIABETES | ABSENT |
| DIABETES | 11 | DIABETIC NEPHROPATHY | PRESENT |
| DIABETES | 12 | DIABETIC RETINOPATHY | PRESENT |
| ... | | | |
| HYPER-TENSION | 20 | HYPERTENSION | ABSENT |
| ... | | | |

*Fig. 10*

CLINICAL ACTION INFORMATION

602, 1102, 1103, 1104, 1101

| SEARCH NUMBER | CLINICAL ACTION CODE | CLINICAL ACTION NAME | CLINICAL ACTION POINT | ... |
|---|---|---|---|---|
| 11 | 1000 | CLINICAL ACTION A | ST1000 | |
| 11 | 2000 | CLINICAL ACTION C | ST2000 | |
| 12 | 3000 | CLINICAL ACTION F | ST3000 | |
| 13 | 1000 | CLINICAL ACTION A | ST1000 | |
| 13 | 2000 | CLINICAL ACTION C | ST2000 | |
| 21 | 1000 | CLINICAL ACTION A | ST1000 | |
| 21 | 1100 | CLINICAL ACTION B | ST1100 | |
| 22 | 1000 | CLINICAL ACTION A | ST1000 | |
| 22 | 1100 | CLINICAL ACTION B | ST1100 | |
| 22 | 2000 | CLINICAL ACTION C | ST2000 | |
| 31 | 1100 | CLINICAL ACTION A | ST1100 | |
| 32 | 1100 | CLINICAL ACTION A | ST1100 | |
| ... | | | | |

*Fig. 11*

CLINICAL ACTION CLASSIFICATION INFORMATION

1002, 1102, 1103, 1201

| INJURY AND ILLNESS CLASSIFICATION | CLINICAL ACTION CODE | CLINICAL ACTION NAME |
|---|---|---|
| DIABETES | 1000 | CLINICAL ACTION A |
| DIABETES | 1100 | CLINICAL ACTION B |
| ... | | |
| HYPERTENSION | 2000 | CLINICAL ACTION C |
| ... | | |

*Fig. 12*

MEDICINE INFORMATION

602 1302 1303 1304 1301

| SEARCH NUMBER | MEDICINE CODE | MEDICINE NAME | MEDICINE POINT | ... |
|---|---|---|---|---|
| 11 | 110 | DIABETES ORAL MEDICINE A | ET110 | |
| 11 | 200 | HYPERTENSION ORAL MEDICINE A | ET200 | |
| 12 | 300 | ANODYNE A | ET300 | |
| 13 | 111 | DIABETES ORAL MEDICINE B | ET111 | |
| 13 | 210 | HYPERTENSION ORAL MEDICINE B | ET210 | |
| 21 | 110 | DIABETES ORAL MEDICINE A | ET110 | |
| 21 | 120 | INSULIN PREPARATION A | ET120 | |
| 22 | 110 | DIABETES ORAL MEDICINE A | ET110 | |
| 22 | 120 | INSULIN PREPARATION A | ET120 | |
| 22 | 200 | HYPERTENSION ORAL MEDICINE A | ET200 | |
| 31 | 121 | INSULIN PREPARATION B | ET121 | |
| 31 | 111 | DIABETES ORAL MEDICINE B | ET111 | |
| 32 | 121 | INSULIN PREPARATION B | ET121 | |
| ... | | | | |

*Fig. 13*

MEDICINE CLASSIFICATION INFORMATION

1002 1302 1303 1401

| INJURY AND ILLNESS CLASSIFICATION | MEDICINE CODE | MEDICINE NAME |
|---|---|---|
| DIABETES | 110 | DIABETES ORAL MEDICINE A |
| DIABETES | 111 | DIABETES ORAL MEDICINE B |
| DIABETES | 120 | INSULIN PREPARATION A |
| DIABETES | 121 | INSULIN PREPARATION B |
| ... | | |
| HYPERTENSION | 200 | HYPERTENSION ORAL MEDICINE A |
| ... | | |

*Fig. 14*

FORMATTED INFORMATION

1501

| 603 | 1502 | 604 | 605 | 1503 | 1504 | | 1505 | | 1506 | | 607 | |
|---|---|---|---|---|---|---|---|---|---|---|---|---|
| HEALTH INSURANCE INSURED PERSON ID | DATA YEAR | SEX | AGE | INJURY AND ILLNESS CODE 10 | INJURY AND ILLNESS CODE 20 | ... | MEDICAL ACTION CODE 1000 | ... | MEDICINE CODE 110 | ... | TOTAL POINT | ... |
| K0001 | 2004 | MALE | 40 | 2 | 2 | | 2 | | 1 | | GT11 | |
| K0002 | 2004 | MALE | 45 | 2 | 1 | | 2 | | 2 | | GT12 | |
| K0003 | 2004 | MALE | 50 | 2 | 0 | | 0 | | 0 | | GT13 | |
| K0004 | 2004 | FEMALE | 50 | 0 | 0 | | 0 | | 0 | | GT21 | |
| ... | | | | | | | | | | | | |

| 1508 | 1509 | 1510 | 1511 | 1512 | 1514 | 1515 | 1516 | |
|---|---|---|---|---|---|---|---|---|
| BMI | ABDOMINAL CIRCUM-FERENCE | FASTING BLOOD SUGAR | SYSTOLIC BLOOD PRESSURE | NEUTRAL FAT | SMOKING | DRINKING | WALKING | ... |
| 25 | 87 | 105 | 130 | 150 | 0 | 100 | 20 | |
| 22 | 70 | 110 | 119 | 90 | 10 | 200 | 10 | |
| 25 | 87 | 110 | 120 | 150 | 0 | 50 | -1 | |
| 20 | 92 | 90 | -1 | 140 | 15 | 0 | 30 | |
| | | | | | | | | |

*Fig. 15*

FORMATTED INFORMATION

603 1502 604 605 1601 1505 1506 607 1501

| HEALTH INSURANCE INSURED PERSON ID | DATA YEAR | SEX | AGE | INJURY AND ILLNESS CODE 10, 20 | ... | MEDICAL ACTION CODE 1000 | ... | MEDICINE CODE 110 | ... | TOTAL POINT | ... |
|---|---|---|---|---|---|---|---|---|---|---|---|
| K0001 | 2004 | MALE | 40 | 4 | | 2 | | 1 | | GT11 | |
| K0002 | 2004 | MALE | 45 | 3 | | 2 | | 2 | | GT12 | |
| K0003 | 2004 | MALE | 50 | 2 | | 0 | | 0 | | GT13 | |
| K0004 | 2004 | FEMALE | 50 | 0 | | 0 | | 0 | | GT21 | |
| ... | | | | | | | | | | | |

1508 1509 1510 1511 1512 1514 1515 1516

| BMI | ABDOMINAL CIRCUM-FERENCE | FASTING BLOOD SUGAR | SYSTOLIC BLOOD PRESSURE | NEUTRAL FAT | SMOKING | DRINKING | WALKING | ... |
|---|---|---|---|---|---|---|---|---|
| 25 | 87 | 105 | 130 | 150 | 0 | 100 | 20 | |
| 22 | 70 | 110 | 119 | 90 | 10 | 200 | 10 | |
| 25 | 87 | 110 | 120 | 150 | 0 | 50 | -1 | |
| 20 | 92 | 90 | -1 | 140 | 15 | 0 | 30 | |
| | | | | | | | | |

*Fig. 16*

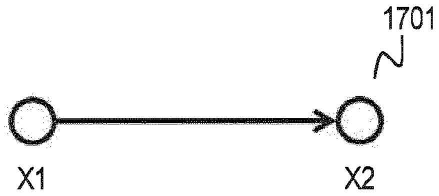


Fig. 17A

1702

| X1=1 | X1=2 | X1=3 |
|---|---|---|
| a1 | a2 | a3 |

Fig. 17B

1703

| | X2=1 | X2=2 | X2=3 |
|---|---|---|---|
| X1=1 | a11 | a12 | a13 |
| X1=2 | a21 | a22 | a23 |
| X1=3 | a31 | a32 | a33 |

Fig. 17C

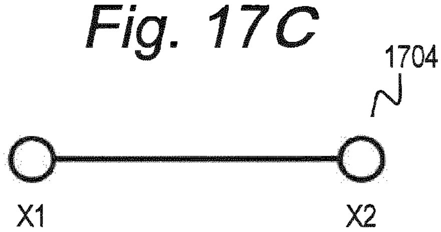


Fig. 17D

1705

| | X2=1 | X2=2 | X2=3 |
|---|---|---|---|
| X1=1 | b11 | b12 | b13 |
| X1=2 | b21 | b22 | b23 |
| X1=3 | b31 | b32 | B33 |

Fig. 17E

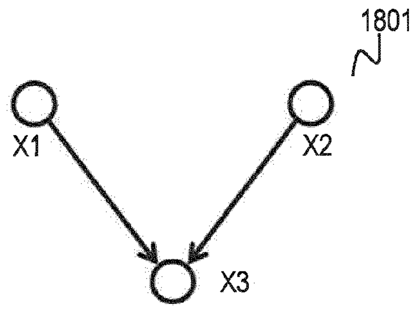


Fig. 18A

1802

P(X1=1)=1/2, P(X1=0)=1/2,
P(X2=1)=1/2, P(X2=0)=1/2,
P(X3=1| X1=0, X2=0) = 0, P(X3=0| X1=0, X2=0) = 1,
P(X3=1| X1=1, X2=0) = 1/3, P(X3=0| X1=0, X2=0) = 2/3,
P(X3=1| X1=0, X2=1) = 2/3, P(X3=0| X1=0, X2=1) = 1/3,
P(X3=1| X1=1 X2=1) = 1, P(X3=0| X1=1, X2=1) = 0

Fig. 18B

1803

| DATA | X1 | X2 | X3 |
|---|---|---|---|
| Case 1 | 0 | 0 | 0 |
| Case 2 | 1 | 0 | 0 |
| Case 3 | 1 | 0 | 1 |
| Case 4 | 0 | 1 | 0 |
| Case 5 | 0 | 1 | 1 |
| Case 6 | 1 | 1 | 1 |
| Case 7 | 1 | 1 | 1 |
| Case 8 | 1 | 0 | 0 |
| Case 9 | 0 | 1 | 1 |
| Case 10 | 0 | 0 | 0 |
| ⋮ | | | |

Fig. 18C

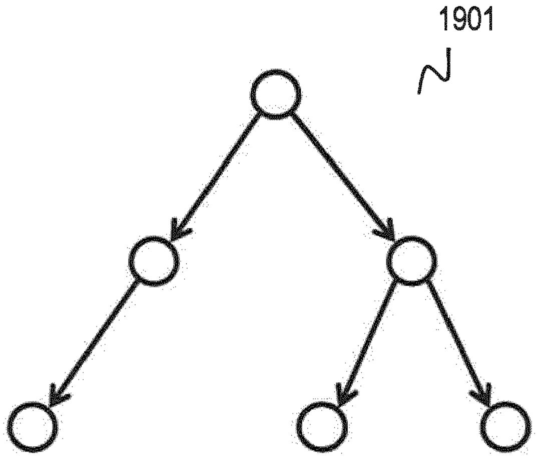


Fig. 19A

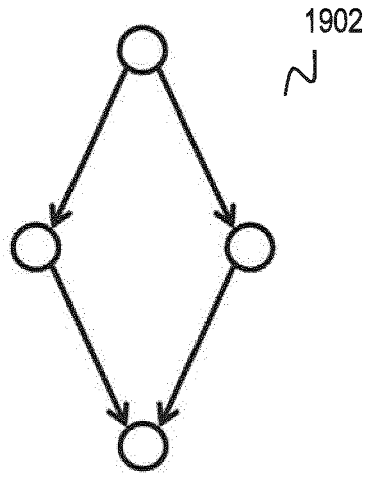


Fig. 19B

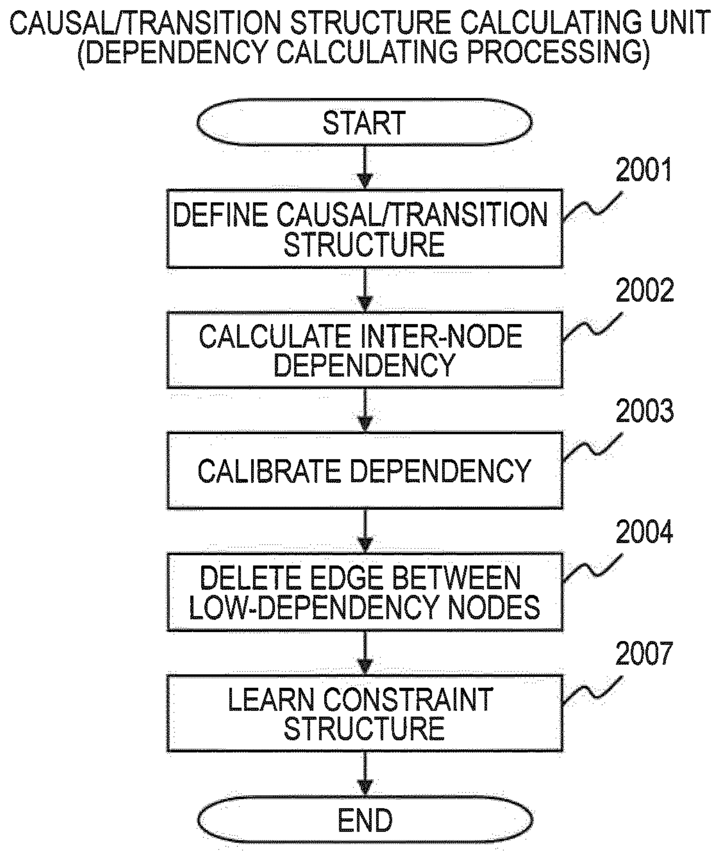


*Fig. 20A*

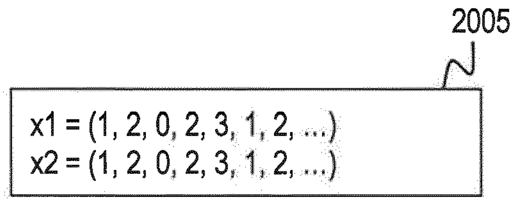


*Fig. 20B*

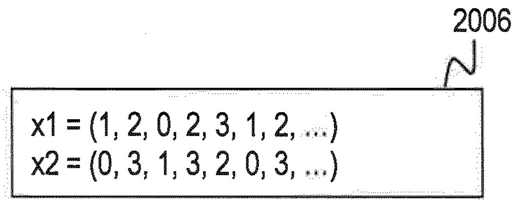


*Fig. 20C*

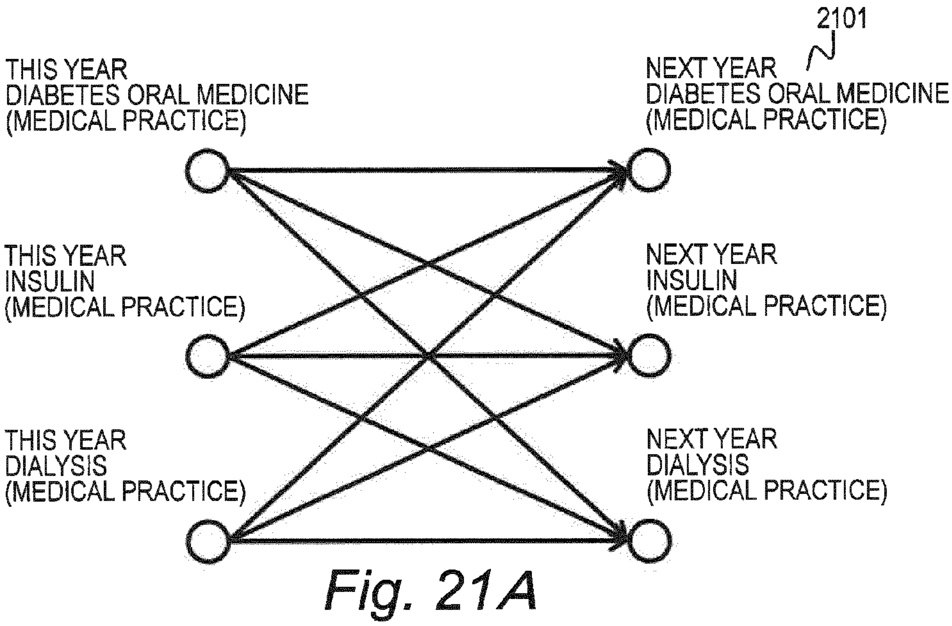


*Fig. 21A*

2102

| HEALTH INSURANCE INSURED PERSON ID (603) | DATA YEAR (1502) | SEX (604) | AGE (605) | DIABETES ORAL MEDICINE (2103) | INSULIN (2104) | DIALYSIS (2105) | ... |
|---|---|---|---|---|---|---|---|
| K0001 | 2004 | MALE | 40 | 1 | 3 | 4 | |
| K0002 | 2004 | MALE | 45 | 2 | 1 | 0 | |
| K0003 | 2004 | MALE | 50 | 3 | 0 | 0 | |
| K0004 | 2004 | FEMALE | 50 | 0 | 0 | 0 | |
| ... | | | | | | | |

*Fig. 21B*

2106

| HEALTH INSURANCE INSURED PERSON ID (603) | DATA YEAR (1502) | SEX (604) | AGE (605) | DIABETES ORAL MEDICINE (2107) | INSULIN (2108) | DIALYSIS (2109) | ... |
|---|---|---|---|---|---|---|---|
| K0001 | 2005 | MALE | 41 | 2 | 5 | 5 | |
| K0002 | 2005 | MALE | 46 | 2 | 2 | 0 | |
| K0003 | 2005 | MALE | 51 | 4 | 0 | 0 | |
| K0004 | 2005 | FEMALE | 51 | 0 | 0 | 0 | |
| ... | | | | | | | |

*Fig. 21C*

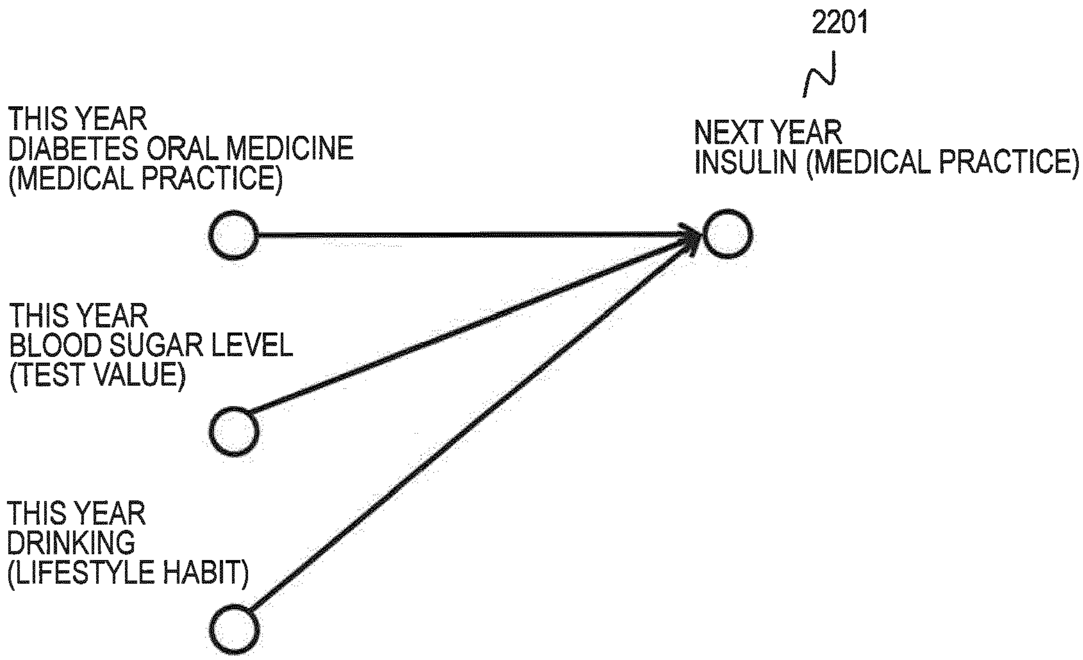


*Fig. 22A*

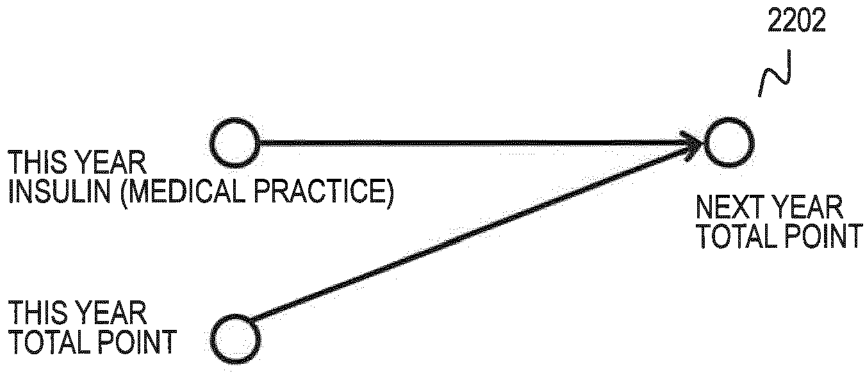


*Fig. 22B*

2301

| PARENT/CHILD | TOTAL POINT OF NEXT YEAR | MEDICAL PRACTICE OF NEXT YEAR | TEST VALUE OF NEXT YEAR | LIFESTYLE HABIT OF NEXT YEAR | TOTAL POINT OF THIS YEAR | MEDICAL PRACTICE OF THIS YEAR | TEST VALUE OF THIS YEAR | LIFESTYLE HABIT OF THIS YEAR |
|---|---|---|---|---|---|---|---|---|
| TOTAL POINT OF NEXT YEAR | | | | | | | | |
| MEDICAL PRACTICE OF NEXT YEAR | CAUSE | | | | | | | |
| TEST VALUE OF NEXT YEAR | | | | | | | | |
| LIFESTYLE HABIT OF NEXT YEAR | | | | | | | | |
| TOTAL POINT OF THIS YEAR | TRANSITION | | | | | | | |
| MEDICAL PRACTICE OF THIS YEAR | CAUSE | TRANSITION | | | | | | |
| TEST VALUE OF THIS YEAR | | CAUSE | | | | | | |
| LIFESTYLE HABIT OF THIS YEAR | | CAUSE | | | | | | |

*Fig. 23A*

2302

| PARENT/CHILD | TOTAL POINT OF NEXT YEAR | MEDICAL PRACTICE OF NEXT YEAR | TEST VALUE OF NEXT YEAR | LIFESTYLE HABIT OF NEXT YEAR | TOTAL POINT OF THIS YEAR | MEDICAL PRACTICE OF THIS YEAR | TEST VALUE OF THIS YEAR | LIFESTYLE HABIT OF THIS YEAR |
|---|---|---|---|---|---|---|---|---|
| TOTAL POINT OF NEXT YEAR | | | | | | | | |
| MEDICAL PRACTICE OF NEXT YEAR | CAUSE | CAUSE | | | | | | |
| TEST VALUE OF NEXT YEAR | | | | | | | | |
| LIFESTYLE HABIT OF NEXT YEAR | | | | | | | | |
| TOTAL POINT OF THIS YEAR | TRANSITION | | | | | | | |
| MEDICAL PRACTICE OF THIS YEAR | CAUSE | TRANSITION | | | | | | |
| TEST VALUE OF THIS YEAR | | | TRANSITION | | | | | |
| LIFESTYLE HABIT OF THIS YEAR | | | CAUSE | | | | | |

*Fig. 23B*

2303

| PARENT/CHILD | TOTAL POINT OF NEXT YEAR | MEDICAL PRACTICE OF NEXT YEAR | TEST VALUE OF NEXT YEAR | LIFESTYLE HABIT OF NEXT YEAR | TOTAL POINT OF THIS YEAR | MEDICAL PRACTICE OF THIS YEAR | TEST VALUE OF THIS YEAR | LIFESTYLE HABIT OF THIS YEAR |
|---|---|---|---|---|---|---|---|---|
| TOTAL POINT OF NEXT YEAR | | | | | | | | |
| MEDICAL PRACTICE OF NEXT YEAR | CAUSE | | | | | | | |
| TEST VALUE OF NEXT YEAR | | CAUSE | | | | | | |
| LIFESTYLE HABIT OF NEXT YEAR | | | | | | | | |
| TOTAL POINT OF THIS YEAR | TRANSITION | | | | | | | |
| MEDICAL PRACTICE OF THIS YEAR | CAUSE | TRANSITION | | | | | | |
| TEST VALUE OF THIS YEAR | | CAUSE | TRANSITION | | | | | |
| LIFESTYLE HABIT OF THIS YEAR | | CAUSE | CAUSE | | | | | |

*Fig. 23C*

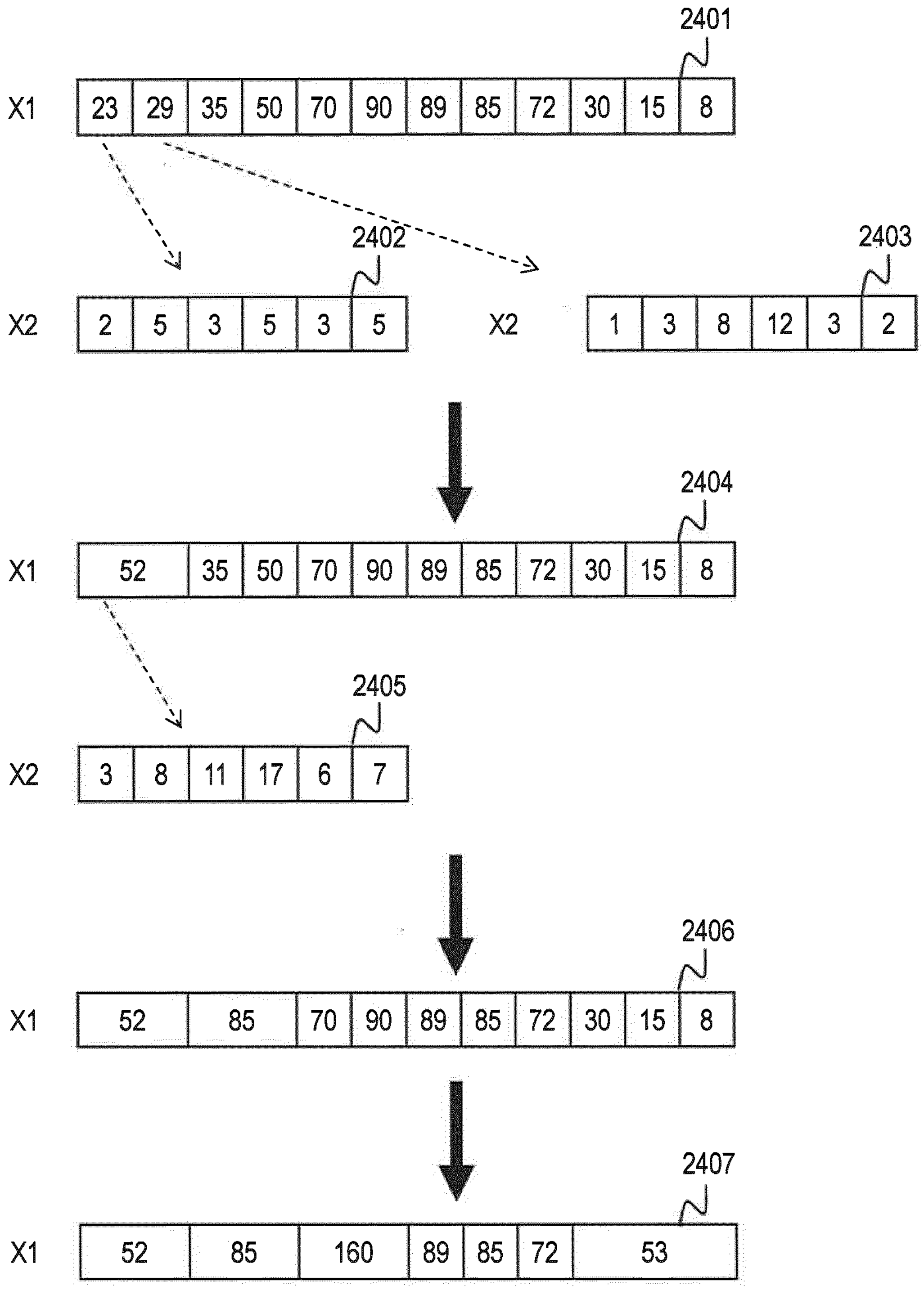
2401
X1
23
29
35
50
70
90
89
85
72
30
15
8
2402
X2
2
5
3
5
3
5
2403
X2
1
3
8
12
3
2
2404
X1
52
35
50
70
90
89
85
72
30
15
8
2405
X2
3
8
11
17
6
7
2406
X1
52
85
70
90
89
85
72
30
15
8
2407
X1
52
85
160
89
85
72
53

Fig. 24

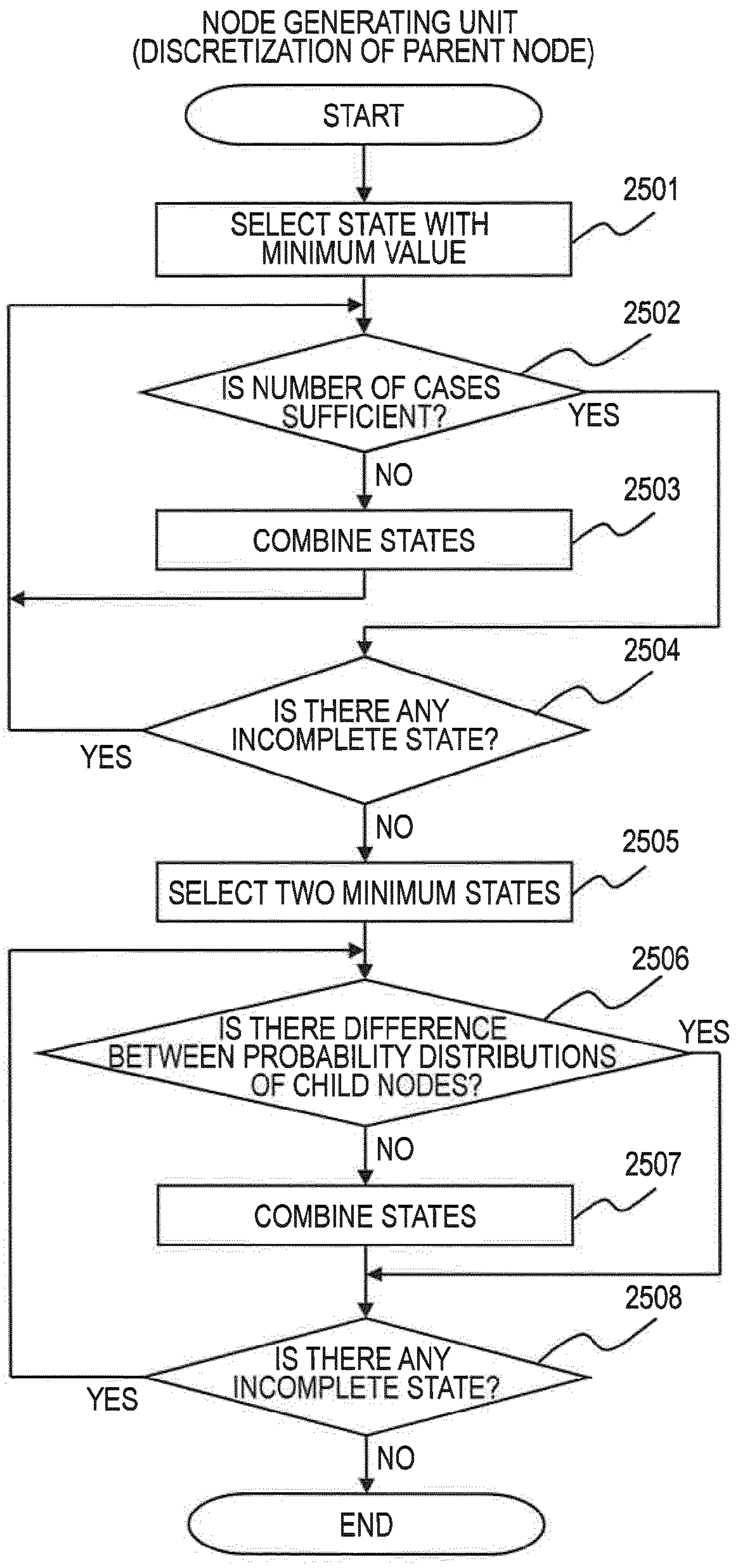
NODE GENERATING UNIT
(DISCRETIZATION OF PARENT NODE)
START
2501
SELECT STATE WITH MINIMUM VALUE
2502
IS NUMBER OF CASES SUFFICIENT?
YES
NO
2503
COMBINE STATES
2504
IS THERE ANY INCOMPLETE STATE?
YES
NO
2505
SELECT TWO MINIMUM STATES
2506
IS THERE DIFFERENCE BETWEEN PROBABILITY DISTRIBUTIONS OF CHILD NODES?
YES
NO
2507
COMBINE STATES
2508
IS THERE ANY INCOMPLETE STATE?
YES
NO
END

Fig. 25

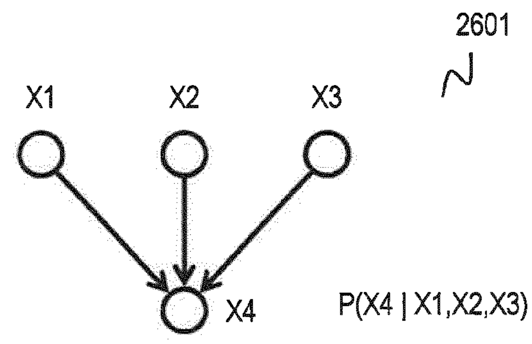


Fig. 26A

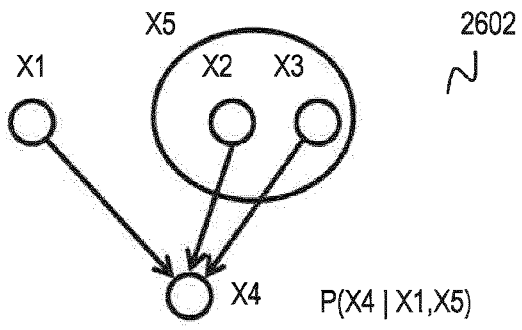


Fig. 26B

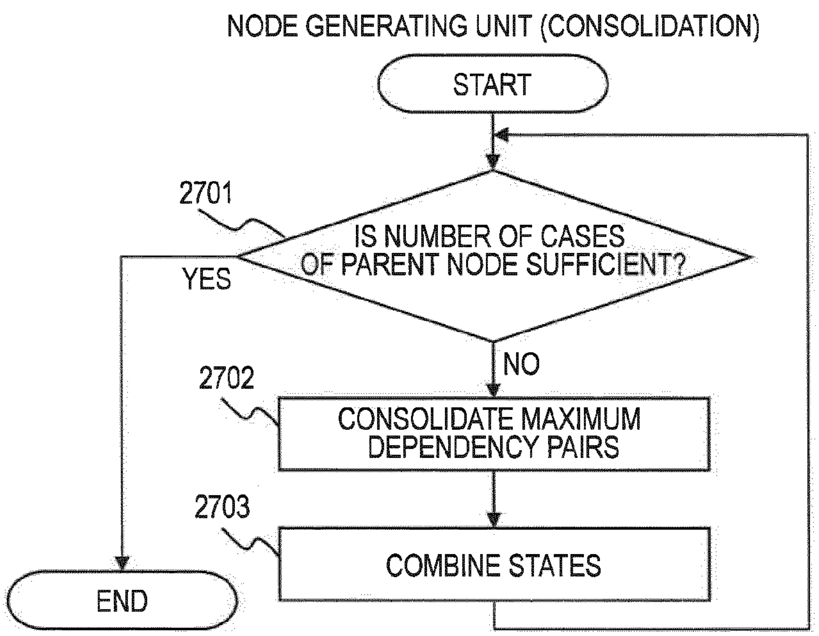


Fig. 27

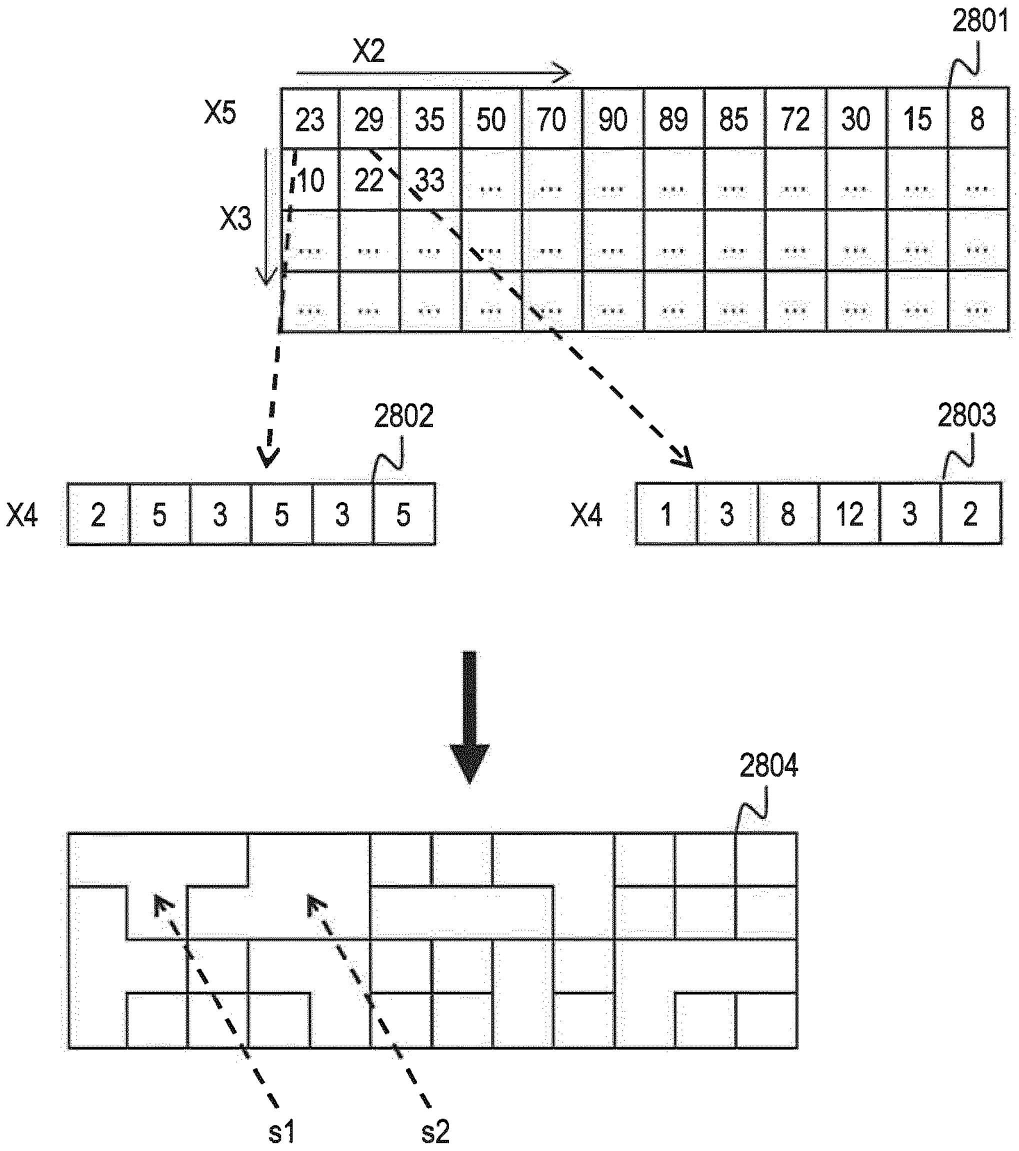
2801
X2
X5
23
29
35
50
70
90
89
85
72
30
15
8
10
22
33
X3
2802
2803
X4
2
5
3
5
3
5
X4
1
3
8
12
3
2
2804
s1
s2

Fig. 28

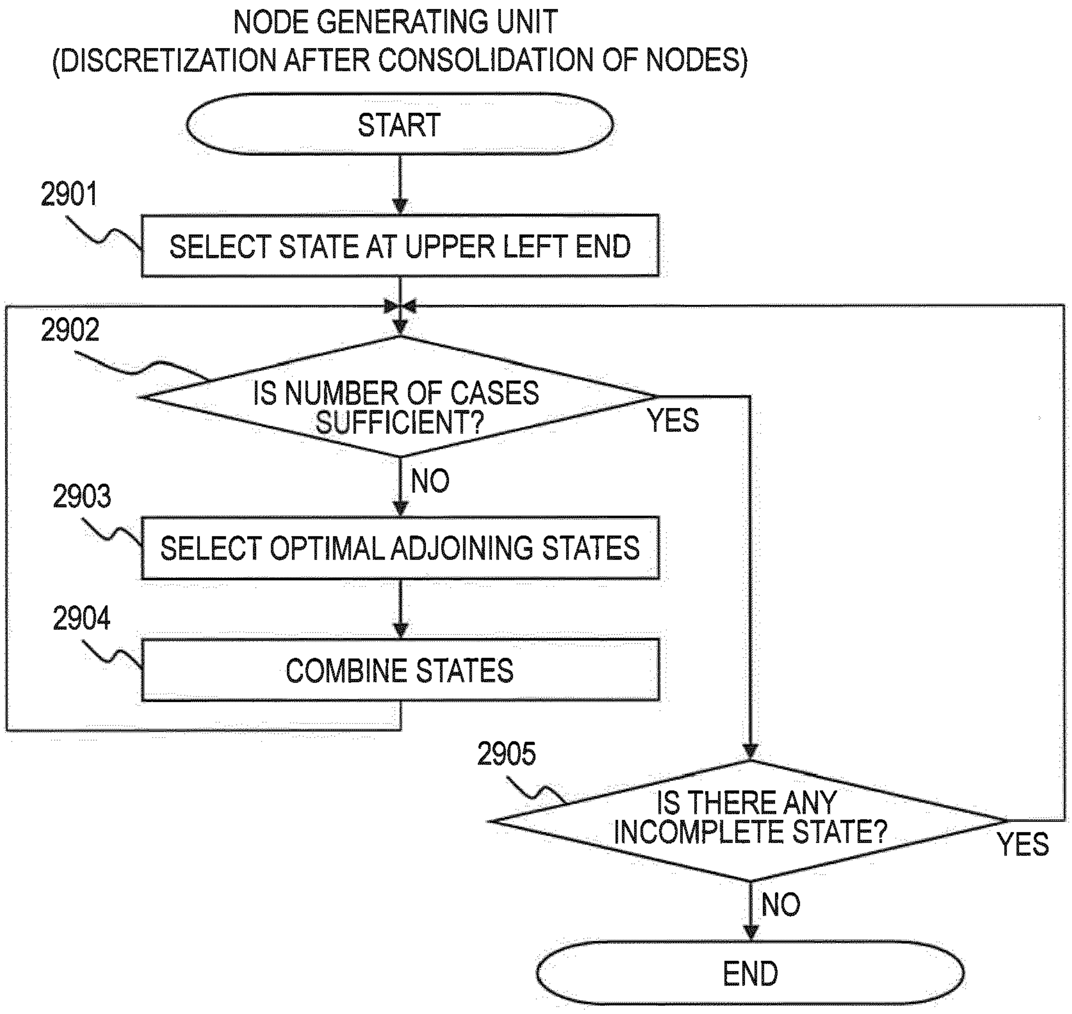
NODE GENERATING UNIT
(DISCRETIZATION AFTER CONSOLIDATION OF NODES)
START
2901
SELECT STATE AT UPPER LEFT END
2902
IS NUMBER OF CASES SUFFICIENT?
YES
NO
2903
SELECT OPTIMAL ADJOINING STATES
2904
COMBINE STATES
2905
IS THERE ANY INCOMPLETE STATE?
YES
NO
END

Fig. 29

3001

| NODE | STATE COMBINATION INFORMATION | | | |
|---|---|---|---|---|
| BMI | 18.01～20.00 | 20.01～21.00 | ... | ... |
| BLOOD SUGAR LEVEL | ... | ... | ... | ... |
| ... | ... | ... | ... | ... |
| | | | | |
| | | | | |

Fig. 30A

3002

| CONSOLIDATED NODE | NODE TO BE CONSOLIDATED | | | |
|---|---|---|---|---|
| CONSOLIDATING NODE 1 | HEIGHT | WEIGHT | ... | ... |
| CONSOLIDATING NODE 2 | ... | ... | ... | ... |
| ... | ... | ... | ... | ... |

Fig. 30B

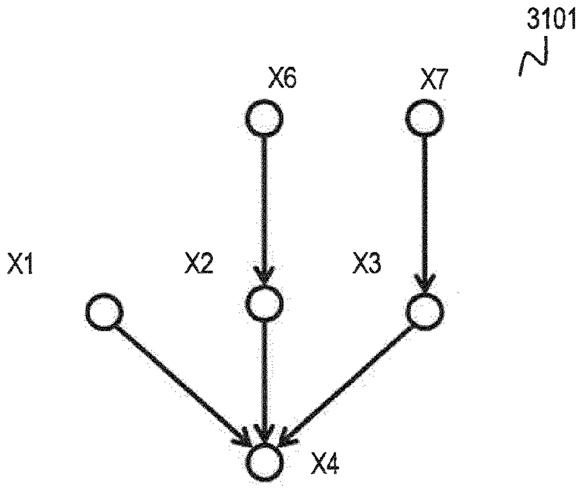


Fig. 31A

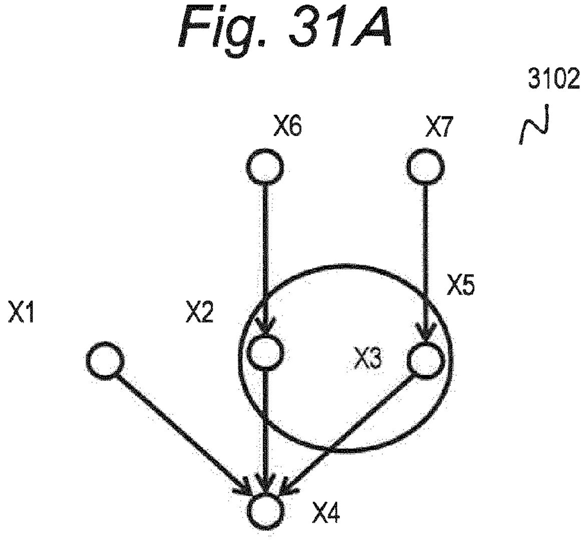


Fig. 31B

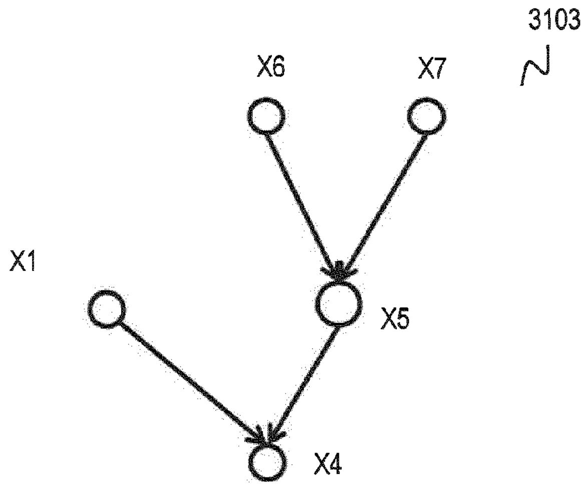


Fig. 31C

3201

| NODE | PARENT NODE | | | |
|---|---|---|---|---|
| X1 | | | | |
| X4 | X1 | X5 | | |
| X5 | X6 | X7 | | |
| X6 | | | | |
| X7 | | | | |

*Fig. 32*

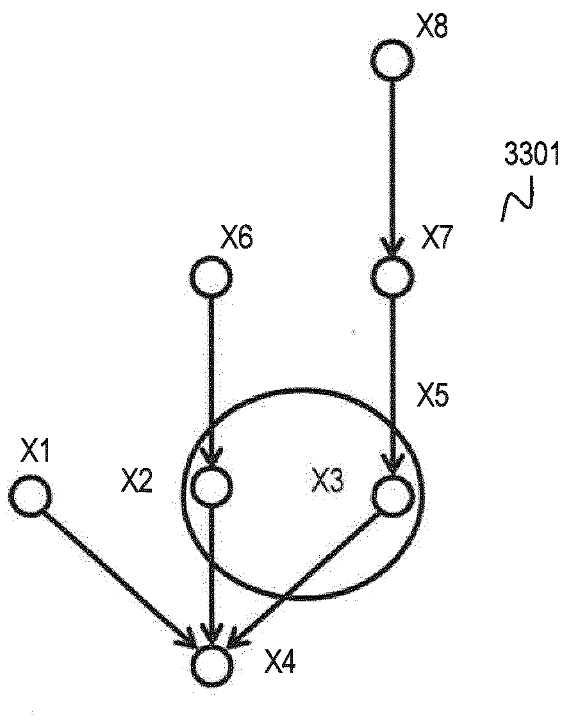


*Fig. 33A*

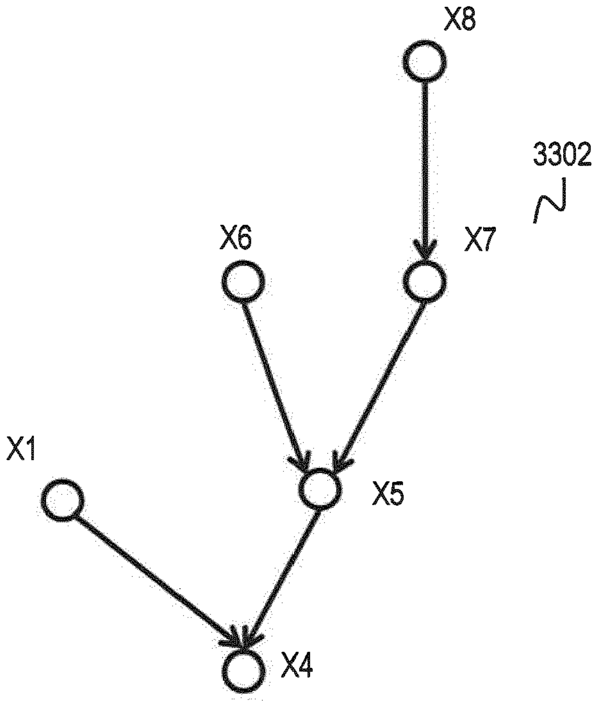


Fig. 33B

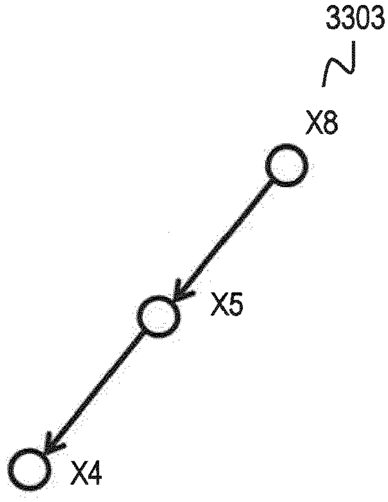


Fig. 33C

3304

P(X1,X4,X5,X6,X7,X8)
=P(X1)P(X6)P(X8)P(X7|X8)P(X5|X6,X7)P(X4|X1,X5)

Fig. 33D

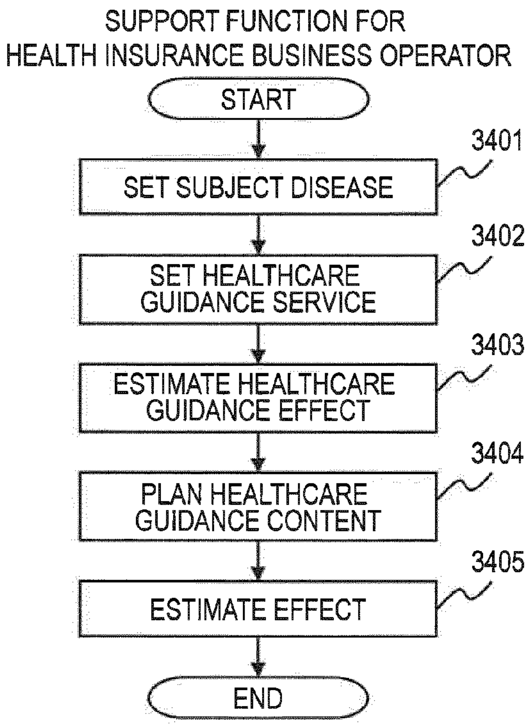


*Fig. 34A*

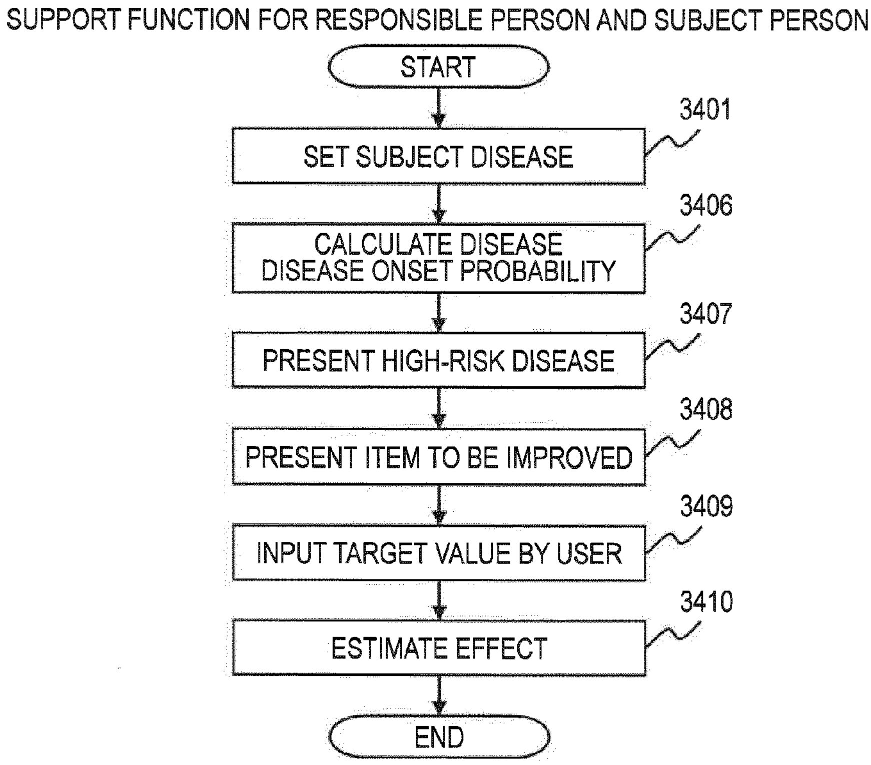


*Fig. 34B*

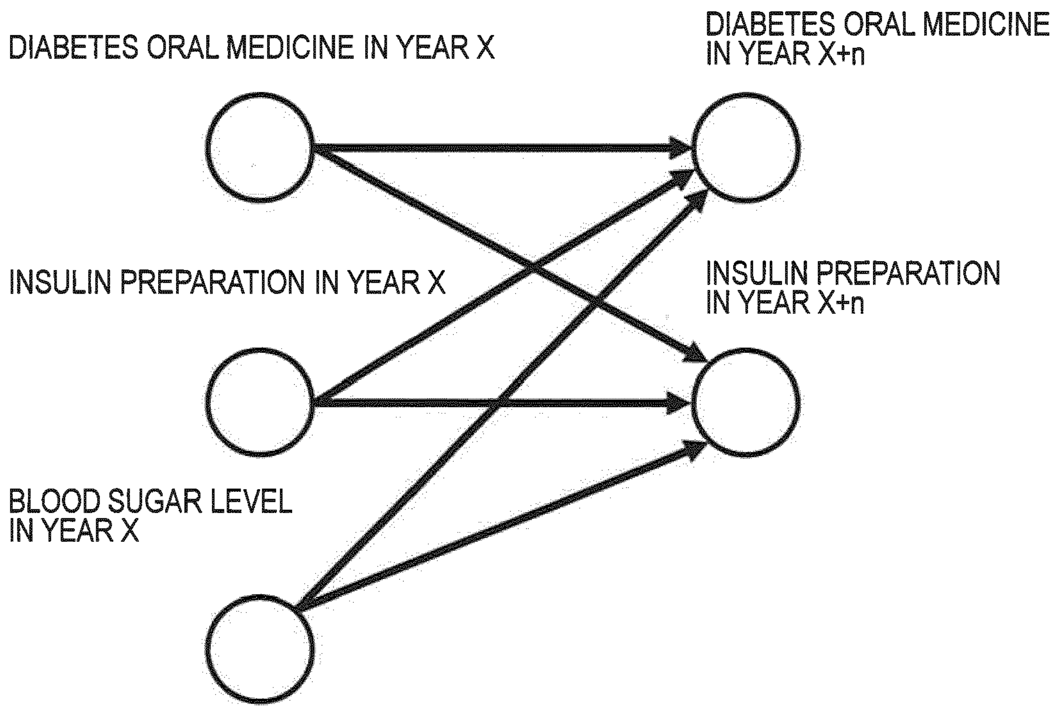
DIABETES ORAL MEDICINE IN YEAR X
INSULIN PREPARATION IN YEAR X
BLOOD SUGAR LEVEL IN YEAR X
DIABETES ORAL MEDICINE IN YEAR X+n
INSULIN PREPARATION IN YEAR X+n

*Fig. 35*

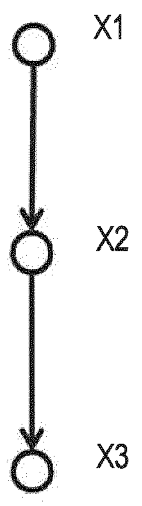
X1
X2
X3

*Fig. 36*

3701

| NUMBER | X1 | X2 | X3 | X4 | X5 | ▪▪▪ |
|---|---|---|---|---|---|---|
| 00001 | 0 | 22 | 87 | 3 | 130 | ▪▪▪ |
| 00002 | 2 | 23 | 84 | 1 | 125 | ▪▪▪ |
| 00003 | 1 | 35 | 70 | 2 | 119 | ▪▪▪ |
| ▪▪▪ | ▪▪▪ | ▪▪▪ | ▪▪▪ | ▪▪▪ | ▪▪▪ | ▪▪▪ |

*Fig. 37A*

3702

| NUMBER | X1 | X2 | X3 | X4 | X5 | ▪▪▪ |
|---|---|---|---|---|---|---|
| 10001 | 0 | 22 | 87 | ? | ? | ▪▪▪ |

*Fig. 37B*

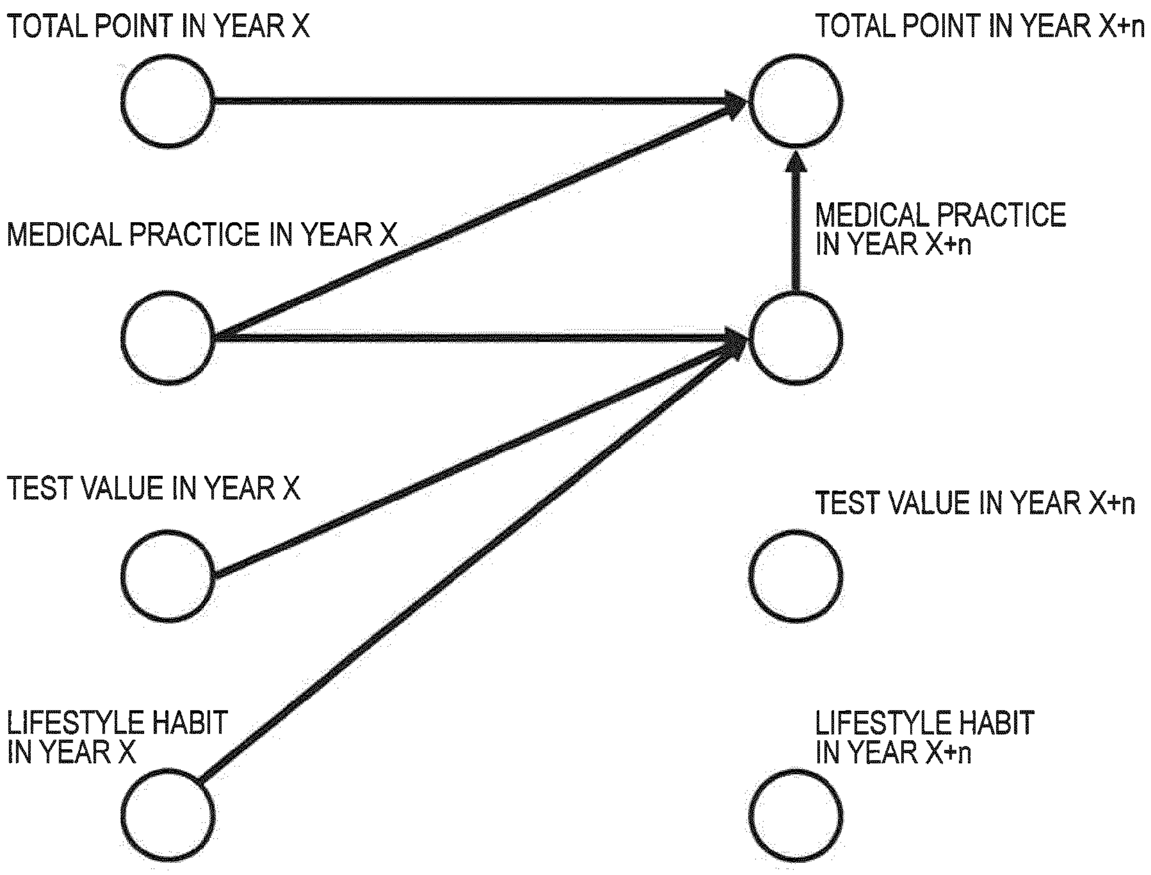
TOTAL POINT IN YEAR X
TOTAL POINT IN YEAR X+n
MEDICAL PRACTICE IN YEAR X
MEDICAL PRACTICE IN YEAR X+n
TEST VALUE IN YEAR X
TEST VALUE IN YEAR X+n
LIFESTYLE HABIT IN YEAR X
LIFESTYLE HABIT IN YEAR X+n

Fig. 38A

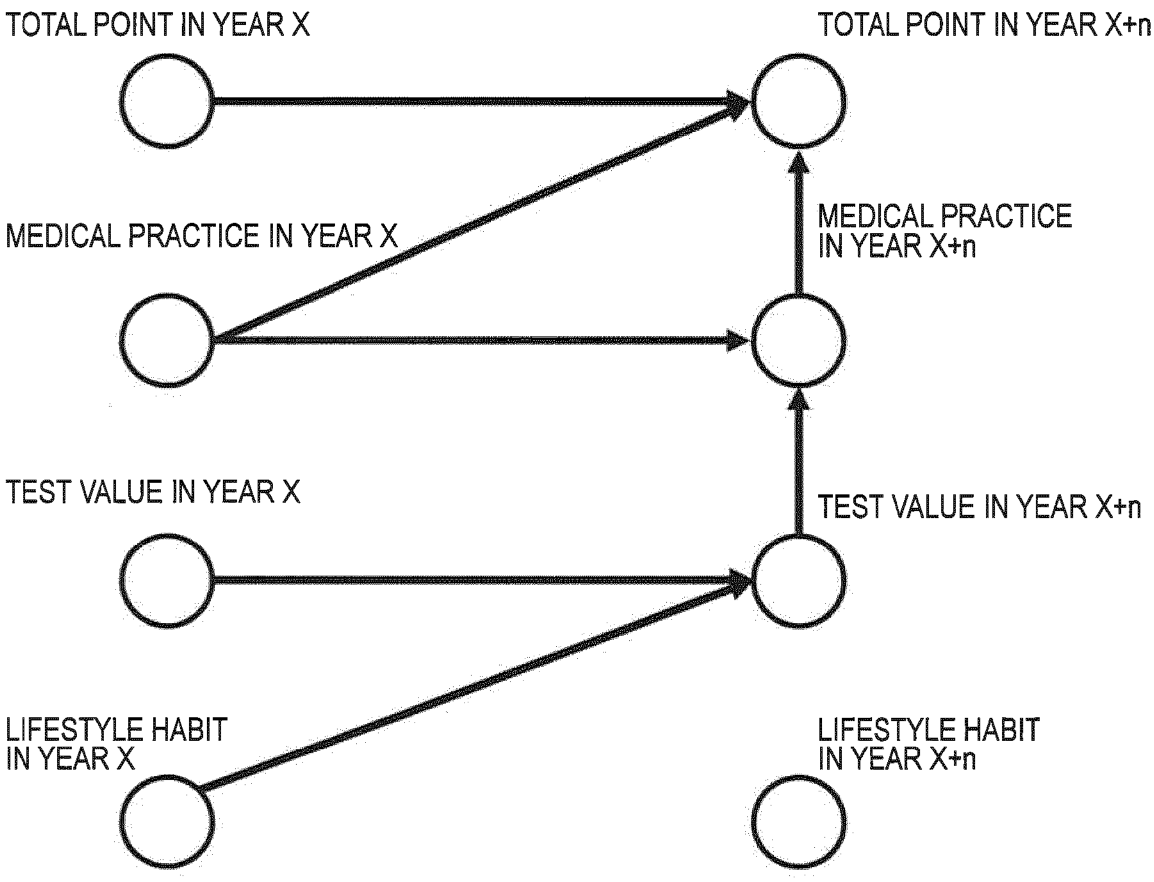
TOTAL POINT IN YEAR X
TOTAL POINT IN YEAR X+n
MEDICAL PRACTICE IN YEAR X
MEDICAL PRACTICE IN YEAR X+n
TEST VALUE IN YEAR X
TEST VALUE IN YEAR X+n
LIFESTYLE HABIT IN YEAR X
LIFESTYLE HABIT IN YEAR X+n

*Fig. 38B*

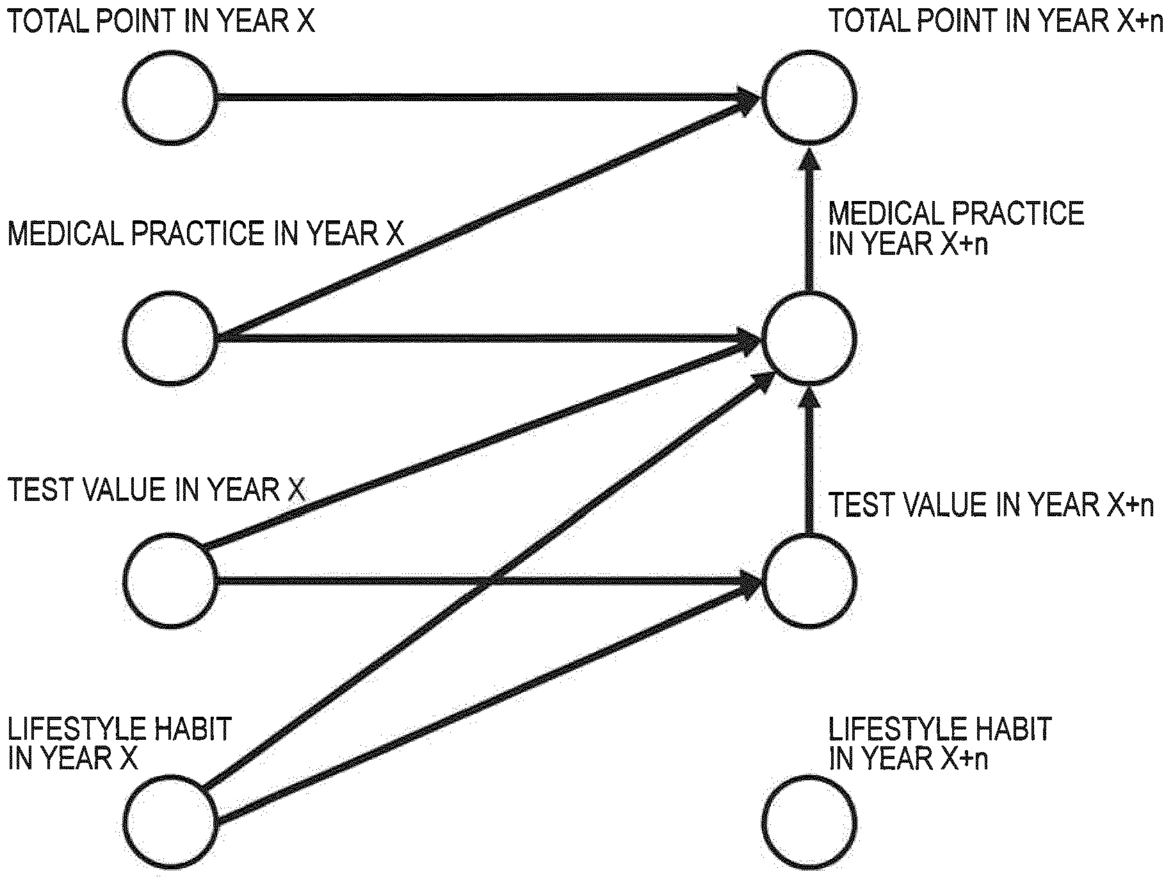
TOTAL POINT IN YEAR X
TOTAL POINT IN YEAR X+n
MEDICAL PRACTICE IN YEAR X
MEDICAL PRACTICE IN YEAR X+n
TEST VALUE IN YEAR X
TEST VALUE IN YEAR X+n
LIFESTYLE HABIT IN YEAR X
LIFESTYLE HABIT IN YEAR X+n

*Fig. 38C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013104664 A
- JP 2012128670 A